# EUROPEAN PATENT APPLICATION

(11) **EP 2 775 412 A1**
(43) Date of publication of application: **10.09.2014**
(21) Application number: 13158164.7
(22) Date of filing: 07.03.2013
(51) Int. Cl.: G06F 19/00

(54) **Method of generating a medical suggestion as a support in medical decision making**

(71) Applicant: Medesso GmbH, 55127 Mainz (DE)
(72) Inventor: Hägele, Michael, 83673 Bichl (DE); Heimann, Dierk, 55124 Mainz (DE); Leidenberger, Freimut, 22453 Hamburg (DE)
(74) Representative: Bühler, Dirk

(57) **Abstract**

A method for generating a medical suggestion useful for supporting a process of medical decision making is presented. A database with a particular, advantageous structure and content allows for the efficient evaluation of received known medical facts based on a set based processing and calculation. In particular, the database may be a relational database. Thus, a digital, automatic, and holistic method generating a medical suggestion is provided, which increases the reliability of the selected medical suggestion which is provided to the user as most probable. The structure of the herein provided database provides for maintenance advantages of the database as the complexity is reduced and single structures of the database are manageable and easily understandable. A corresponding medical decision support system is presented as well.

## Description

### FIELD OF THE INVENTION

The present invention relates to the provision of information useful in medical decision making. In particular, the present invention relates to a method of generating a medical suggestion, a program element for generating a medical suggestion, a computer-readable medium, in which a computer program for generating a medical suggestion is stored, and to a medical decision support system for generating a medical suggestion.

### BACKGROUND OF THE INVENTION

In average, medical knowledge is doubling every four years. Consequently, it is simply impossible for patients and even a demanding task for health professionals to keep track of all relevant aspects during medical decision making. Different physical, biochemical, and information technology (IT) based solutions have been developed over decades to assist and support medical doctors during the process of medical decision making. However, such medical technology devices like for example a computer tomograph or an ultrasonic based imaging device merely provide the clinician with additional data, but can hardly take into account medical knowledge. Therefore, reliable medical decisions currently require that the received computer tomography images or ultrasonic images are combined with the knowledge of the medical doctor and/or are combined with knowledge provided by specialist literature. Moreover, the medical doctor needs to rely on an interaction with the patient in order to be provided with the necessary and sufficient information for the decision making.

Unfortunately, highly structured data are currently not available in sector of health care. Further, prior art IT systems for medical support are based on tree diagrams which are supposed to reflect medical relationships according to the present medical knowledge. However, it may be a too complex task for a tree diagram based system to take interrelation into account. For example, a patient may not only suffer from one disease A but may have diseases A and B together. The therapy suggestion, as an embodiment of the medical suggestion MSᵢ, for the combination A and B can be quite different from the sum of the individual treatments of diseases A and B.

Moreover, the process of generating an individual medical finding by a medical doctor based on a laboratory letter is time-intensive, tiresome, and relatively cost-intensive. Depending on the medical topic, profound medical expertise and a large amount of experience is necessary. For example, in gynaecological endocrinology, only a small amount of relatively expensive medical specialists are available, which are able to accurately evaluate a complete set of lab parameters, as said lab parameters are highly dependent from a large variety of factors. One and the same value of a lab parameter may be interpreted as being normal once, whereas it can be highly pathologic in case additionally known medical facts like age, day of the cycle, status of pregnancy, week of pregnancy, and/or phase of live are taken into account.

### SUMMARY OF THE INVENTION

Consequently, there may be a need for improving the provision of support during the process of medical decision making. It may thus be seen as an object of the present invention to provide for an improved support for medical decision making.

The object is solved by the subject-matter of the independent claims. Further exemplary embodiments and advantages of the present invention are comprised by the dependent claims.

The following detailed description of the present invention similarly pertains to the method of generating a medical suggestion, the program element for generating a medical suggestion, the computer readable medium, and the medical decision support system. In other words, synergetic effects may arise from different combinations of the embodiments, although they might not be described hereinafter explicitly. Moreover, any reference signs in the claims should not be construed as limiting the scope of the claims.

Before the invention is described in detail with respect to some of its preferred embodiments, the following general definitions are provided.

The present invention as illustratively described in the following may suitably be practiced in the absence of any element or elements, limitation or limitations, not specifically disclosed herein.

The present invention will be described with respect to particular embodiments and with reference to certain figures, but the invention is not limited thereto, but only by the claims.

Whether the term "comprising" is used in the present description and claims, it does not exclude other elements. For the purpose of the present invention, the term "consisting of' is considered to be a preferred embodiment of the term "comprising of". If hereinafter a group is defined to comprise at least a certain number of embodiments, this is also to be understood to disclose a group, which preferably consists only of these embodiments.

Where an indefinite or definite article is used when referring to a singular noun, e. g. "a", "an", or "the", this includes a plurality of that noun, unless something else is specifically stated hereinafter. The terms "about" or "approximately" in the context of the present invention denote an interval of accuracy that the person skilled in the art will understand to still ensure the technical effect of the feature in question. The term "typically" indicates deviation from the indicated numerical value of plus/minus 20 percent, preferably plus/minus 15 percent, more preferably plus/minus 10 percent, and even more preferably plus/minus 5 percent.

Technical terms are used herein by their common sense. If a specific meaning is conveyed to certain terms, definitions of terms will be given in the following in the context of which the terms are used.

The term "database" shall be understood as a digital entity on which data and/or information regarding medical knowledge or medical correlations/associations can be stored. In particular, the database shall be understood as a data storage on which the herein described associations or correlations between medical suggestions MSᵢ and medical facts Fⱼ as well as between medical suggestions and weights W_{i,j} can be stored. In particular, the database may be embodied as a single physical unit, for example on a single server, however, the database may be distributed over a plurality of servers and/or over a plurality of data storage devices, and may be accessed via a network system. Consequently the present invention may also be used within a cluster of servers, amongst which the herein described database is distributed. Several different aspects regarding an advantageous generation of such a database will be provided hereinafter in detail. Further, if desired, the database provides for a structure such that all medical facts Fⱼ are autarkic and equivalent.

Moreover, the database may be embodied as a relational database. In particular, a relational database facilitates set operations, like calculations and selections as described herein. In this context, SQL-instructions may be used by the present invention. Therein, SQL stands for structured query language and is a special-purpose programming language designed for managing data held in a relational database management systems (RDBMS). However, if desired, also other programming language can be used without departing from the present invention.

In the context of the present invention the terms "autarkic" and "equivalent" may be understood as follows. Each of the medical facts can be submitted independently as an input in the sense that they do not need to have a relation amongst each other. In other words, each medical fact Fⱼ stands for itself and can influence with different weights different medical suggestions. In exemplary cases a medical fact in combination with one or more other medical facts is attributed with different weights. However, this does not exclude that there can be different medical facts, or combinations of said medical fact with others, which achieve or are attributed to the same weight.

The term "to be associated" will be used in the context of the medical suggestions MSᵢ, the medical facts, Fⱼ, and the weights W_{i,j} as follows. In general, in case an association between a medical suggestion MSᵢ and a medical fact Fⱼ, is comprised by the database, the database comprises or defines a relationship or link between said medical suggestion MSᵢ and said medical fact Fⱼ. The same holds true for the association between a medical fact Fⱼ of a medical suggestion MSᵢ with a weight W_{i,j}. In particular, a medical suggestion MSᵢ which is associated in the database with a medical fact Fⱼ reflects the fact or knowledge of the database, that the medical fact Fⱼ to a certain amount influences or contributes to the medical suggestion MSᵢ. In other words, the medical fact Fⱼ should be taken into account, to a certain weighted amount defined in the database, when the medical suggestion MSᵢ is evaluated in view of or dependent from the received known facts. In other words, the dependency of the medical suggestion MSᵢ from the medical fact Fⱼ is represented or reflected in the database by means of said association. In case of an input comprising different specific values of one (or more) medical fact Fⱼ, the used association structure of the database ensures that each medical suggestion MSᵢ out of the basic set S0 can be identified, which is at least to a certain amount, influenced by said medical facts Fⱼ of the input.

As an exemplary illustrative embodiment, the medical suggestion MSᵢ can be embodied as a folder which comprises at least one or a plurality of medical facts Fⱼ. As an exemplary non-limiting embodiment, the medical suggestion MSᵢ may be the diagnosis of influenza, wherein the associated medical facts Fⱼ comprised by said database may be F₁ = body temperature, F₂= a specific blood parameter, F₃= irritated and watering eyes, and F₄ =fatigue. Correspondingly, the database "knows" that said parameters are correlated with the medical suggestion influenza. However, the underlying principle of this correlation between medical suggestions and medical facts does not only apply for this example of a disease or diagnosis and associated symptoms, but is applied by the present invention in a much broader way, which will be explained in more detail hereinafter. Details about the used weights W_{i,j} will also be explained hereinafter.

Moreover, the term "medical fact Fⱼ" can generally be seen as parameter which is suitable for describing a medical situation. In principle, the medical facts Fⱼ can be used as inputs for the method and the system to calculate the desired result. In the context of the present invention, a medical fact Fⱼ may be embodied in various different ways, like for example a parameter describing the patient like the age or the gender of the patient, a body weight or a given result of a medical finding, or medication data, or an allergy or a result of a function test, or information received from for example a professional questionnaire. Many other embodiments will be given hereinafter. In any case, a medical fact Fⱼ provides for basic or atomic information about an individual patient or circumstances in which a patient lives. The "value or values" of such a medical fact can be seen as "85" beats per minute for the example of the medical fact Fⱼ being the heart beat of the patient. Thus, the value may be seen as a characteristic, markedness or peculiarity of the medical fact Fⱼ at a given point in time or as a time-independent value. As an exemplary embodiment, a medical fact Fⱼ may be seen as an N-type vector in which a time evolvement of the medical fact is comprised. For example, in case the medical fact Fⱼ is embodied as the body weight of the patient in kilograms, the corresponding vector of the medical fact may be provided in the following form: [60 kg (at 15.12.2010) 70 (at 27.2.2010), 75 kg (at 31.12.2010), 73 kg (at 5.1.2011), ...]. Consequently, also time dependent medical facts Fⱼ may be provided and used by the present invention.

Moreover, the term "known facts" shall be seen as data input for the system used by the method of the present invention and are assumed to be true. The received known facts may be provided via data transmission or may be received by the system and may be used by the method after a user has provided a corresponding input. An automatic data transfer from a patient directory or from previous diagnosis or other medical events can be taken into account by the present invention as known facts in form of values of medical facts Fⱼ.

The term "weight W_{i,j}" can be seen as a discrete or continuous probability distribution or function by means of which the dependency or importance of a value of the associated medical fact Fⱼ for the corresponding medical suggestion MSᵢ is expressed. It can thus be seen as a strength of the correlation between a value, characteristic, markedness or peculiarity of the medical fact Fⱼ and the fact that the associated medical suggestion is true or correct in the individual situation. In other words, such a weight W_{i,j} may reflect the probability that, based on the received value of the medical fact Fⱼ, the provision of the medical suggestion MSᵢ to the user as an output is an appropriate and accurate medical measure of the herein described system and/or method. In particular, specific values of said weight W_{i,j} can be positive or negative such that the contribution of one medical fact to the calculated total score of the respective medical suggestion MSᵢ can be positive or negative as well. In an exemplary embodiment, the weight W_{i,j} may be seen as a so-called point system, which attributes specific values or probabilities to the known facts, such that scores of the relevant and selected medical suggestions can be calculated. Such calculation and mathematical embodiments thereof will be explained in more detail hereinafter. Consequently, the medical facts Fⱼ, which are associated with one medical suggestion MSᵢ, can be seen as weighted suggestion components which all, positively or negatively, contribute to the score of said medical suggestion MSᵢ. Thus, the score of said medical suggestion may be seen as a total or overall score which is summed over all associated and received known medical facts Fⱼ.

In general, the herein used weights W_{i,j} may be seen as a function of the respective value or values of the corresponding medical fact Fⱼ or a combination of medical facts. Consequently, the terminology W_{i,j}(Fⱼ) can be used. In other words, the value of the weight function W_{i,j} depends on the actual value of the medical fact Fⱼ. Moreover, the value of the weight W_{i,j} depends on the corresponding medical suggestion MSᵢ. Consequently, weights W_{i,j} may be written in the form of W_{i,j}(MSᵢ, Fⱼ).

The term "medical suggestion MSᵢ" in the context of the present invention can be embodies in various different ways. Exemplary embodiments are a medical diagnosis, a text block, a medical finding, an evaluation of a lab value, a treatment recommendation, patient questionnaire, nutrition suggestion, or a medical question. However, also many other exemplary embodiments are possible and will be explained hereinafter. In general, the medical suggestion can be seen as being defined by the associated medical facts Fⱼ and/or by associated other medical suggestions, e.g. MSₘ or MSₙ. Moreover, the corresponding weight W_{i,j} contributes to the definition of the medical suggestion MSᵢ as well. Furthermore, in general, the medical suggestion may be seen as a medical event, incident, or occurrence. The medical suggestion, as an output of the method or the system of the present invention, is attributed with a score or scores and can be the basis for the user or a device for the further procedure. Thus, it may be seen as a support for the medical decision making process.

Further, the term "generating a medical suggestion" in the context of the present invention can be seen as selecting at least one or a plurality of medical suggestions MSᵢ out of the basic set S0 and calculate a respective score for some or each of said medical suggestion MSᵢ based on associations comprised in the database.

According to an exemplary embodiment of the invention, a method of generating a medical suggestion useful for supporting a process of medical decision making is presented. The method comprises the steps of providing for a database with a basic set S0 of medical suggestions MSᵢ. In said database, at least some of the medical suggestions MSᵢ are associated with at least one respective medical fact Fⱼ,. Furthermore, in the database the respective medical fact Fⱼ of the at least some medical suggestions is associated with a weight W_{i,j}. Moreover, the method comprises the step receiving known facts in the form of values of medical facts Fⱼ, which known facts are associated with an individual patient. The step selecting a subset S1 of medical suggestions out of the basic set S0 based on the received known facts is comprised by the method. Furthermore, calculating a respective score for at least some medical suggestions MSᵢ of the subset S1 based on the received values of the medical facts Fⱼ and the respective weight W_{i,j} is comprised by the method.

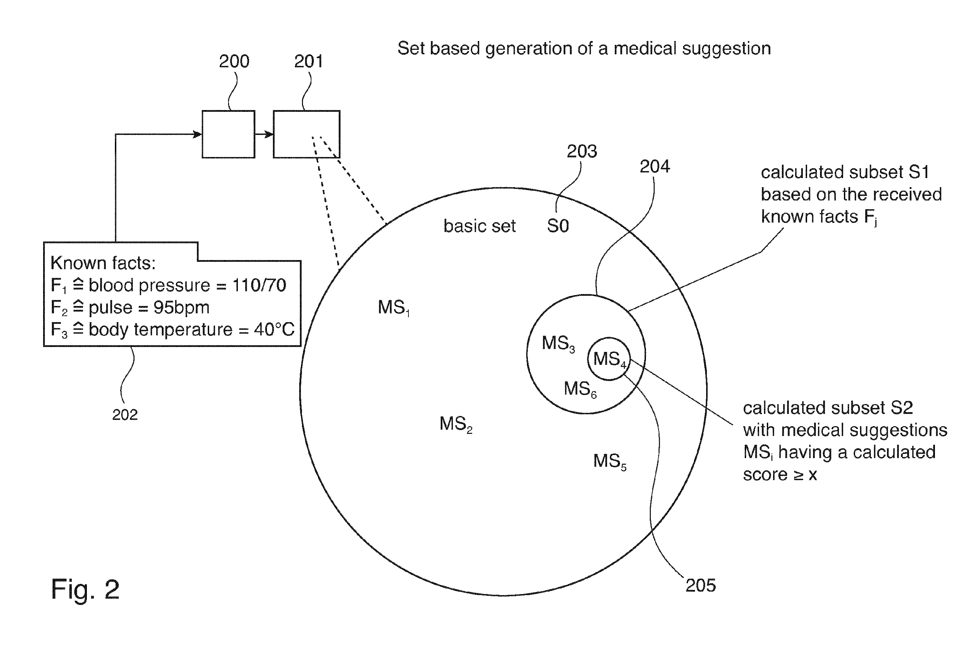

The method of supporting a medical decision making may be implemented in a PC, a server, a calculation unit or may be carried out by distributed computation. This method may be carried out by a medical decision support system, which system will be explained in more detail hereinafter. As has been described before, the presented method is processed and/or carried out on a set basis, which provides for certain advantages over prior art methods which are based on a data structure and/or a database structure in form of tree diagrams. This advantage and further advantages of the set-based generation and calculation of a medical suggestion are described hereinafter, especially in the context of Figs. 2, 3, 4, and 5. As will become apparent from the following explanation, the calculation of the respective scores of the medical suggestions MSᵢ can be carried out and provided to the user in form of a value which reflects a probability that the respective individual medical suggestion MSᵢ is correct and appropriate for further procedure. Based on mathematical correlations stored in the database, the associations between the medical suggestions MSᵢ and the medical facts Fⱼ as well as by means of the associated weights W_{i,j}, a respective score and/or probability of the medical suggestions MSᵢ are calculated by the presented method. If desired, a comparison between the calculated score and a predetermined threshold can be carried out by the presented method. For example, scores between 200 and 600 points are classified as a suspicion, scores between a value of 601 and 950 are classified as probable, whereas scores larger than 950 are classified as highly probable. However, also other classifications with 2, 4, or more classes can be used and also other limitations of the used classes can be applied.

As will become clear from and elucidated with the examples 1 to 5, a database with the herein described advantageous structure and content allows for the efficient evaluation of the received known facts based on a set based processing and calculation. In particular, the database may be a relational database. Thus, a digital, automatic, and holistic method generating a medical suggestion MSᵢ is provided, which increases the reliability of the selected medical suggestion which is provided to the user as most probable. The structure of the herein provided database provides for maintenance advantages of the database as the complexity is reduced and single structures of the database are manageable and easily understandable.

As will become apparent from the following explanation, the present invention allows for concurrently pursuing multiple targets by a holistic approach.

Of course, all medical suggestions MSᵢ of the basic set S0 can be associated with at least one respective medical fact Fⱼ in the database. In particular, associations with a plurality of respective medical facts Fⱼ can be comprised by the database for each medical suggestion MSᵢ. Furthermore, if desired, the step of calculating a respective score can be carried out for each medical suggestion MSᵢ which is comprised by the selected subset S1. However, this may be a user-specific adjustment.

In addition, an output may be generated for the user such that the user is provided with the calculated scores of the medical suggestions MSᵢ of the subset S1 for the consideration of the user. In particular, in an exemplary embodiment the ranking or order of the scores of the medical suggestions MSᵢ out of subset S1 is presented to the user via an interface like for example a display or by means of a generated letter. These aspects and further aspects of the present invention will be discussed in the context of Figs. 2 to 7.

In other words, the presented method provides for decision support for e.g. the patient, the medical doctor or a lab robot and facilitates a navigation towards an improved decision based on an interactive and/or iterative process. Said interactive and/or iterative aspects of the present invention will be described in more detail. Moreover, in this context, an "improvement" is seen in the provision of a medical suggestion with a high probability of correctness. Consequently, the presented method facilitates an increase of the probability level of the medical suggestion provided to the user, and/or reduces the set or results by number. In other words, the presented method provides for a database comprising digitized medical knowledge, and allows for an efficient evaluation of medical suggestions MSᵢ based on the structure of the database and the received known facts. For example, the database may be generated by a group of experts which define the herein described associations between the medical suggestions MSᵢ and the respective medical facts Fⱼ as well as the determination of the corresponding weight W_{i,j}. Consequently, the database as used in the herein presented method comprises digitized medical knowledge stored in a particularly defined way, as described above and below.

During the step of calculating the respective, individual score, the method of an embodiment of the present invention may comprise for the step of summing each value of the weight W_{i,j} for each medical fact Fⱼ associated with said medical suggestion MSᵢ. The summation leads to the score of each medical suggestion MSᵢ which is comprised in the selected subset S1. Consequently, said summation is based on the assumption that a medical suggestion is more probable in case more medical facts Fⱼ indicate or point towards a high probability of said medical suggestion.

However, in another exemplary embodiment the following principle may be used in addition or alternatively for calculating the score of a medical suggestion. In case a medical suggestion comprises/is defined by combinations of medical facts, e.g. medical facts which linked by Boolean Operators as explained for the Examples 1 and 2 described below, only particular combinations may be used for the score calculation of said medical suggestion. In particular, only specific combinations may be used, in which at least a threshold number of the received known facts do play a role. For example, four different medical facts are received as known facts and a medical suggestion is defined by three combinations of said medical facts. Such combinations and the working principle are explained herein in detail, e.g. in the context of the Examples 1 and 2 described below. In the present example, the first and second combinations may make use of two received known facts respectively, whereas the third combination makes use of all four received know facts. In this example, the database may comprise a corresponding condition such that only the score of the third combination is taken into account during said calculating of the medical suggestion. This may be seen as a preferred use of combination of medical facts which make use of a minimum amount of the received known facts. This may provide a specificity criterion for the selection of the combinations of medical facts, comprised by the database, which are used for the score calculation of the medical suggestion. This may provide for an enhanced overview for the user.

As already explained above, the herein presented method involves a set-based calculation process for generating the medical suggestion. The calculation process calculates with possible sets, which comprise medical facts Fⱼ and medical suggestions MSᵢ as components. These components themselves should not be considered as sets, but as objects which from an IT point of view may use self-referencing.

In the database, which may be embodied as a relational database, the calculation process starts to select those medical suggestions MSᵢ with at least one association to said received medical facts based on the received known facts in the form of values of the medical facts Fⱼ. By means of so-called knockout criteria comprised by the database, medical suggestions MSᵢ may be removed from further consideration or may not be selected at all in case said knockout criterion is fulfilled. Moreover, combinations of the received known facts can be evaluated, for example by carrying out Boolean operations which may be comprised/defined in the database, which will be illustratively explained herein. Moreover, as will be explained later on with respect to Examples, a medical suggestion may also be associated with another medical suggestion, such that a medical-suggestion-in-another-medical-suggestion-structure may be comprised in the database. In such a case, first the score of the inner medical suggestion is calculated, based on which the score of the outer medical suggestion is calculated.

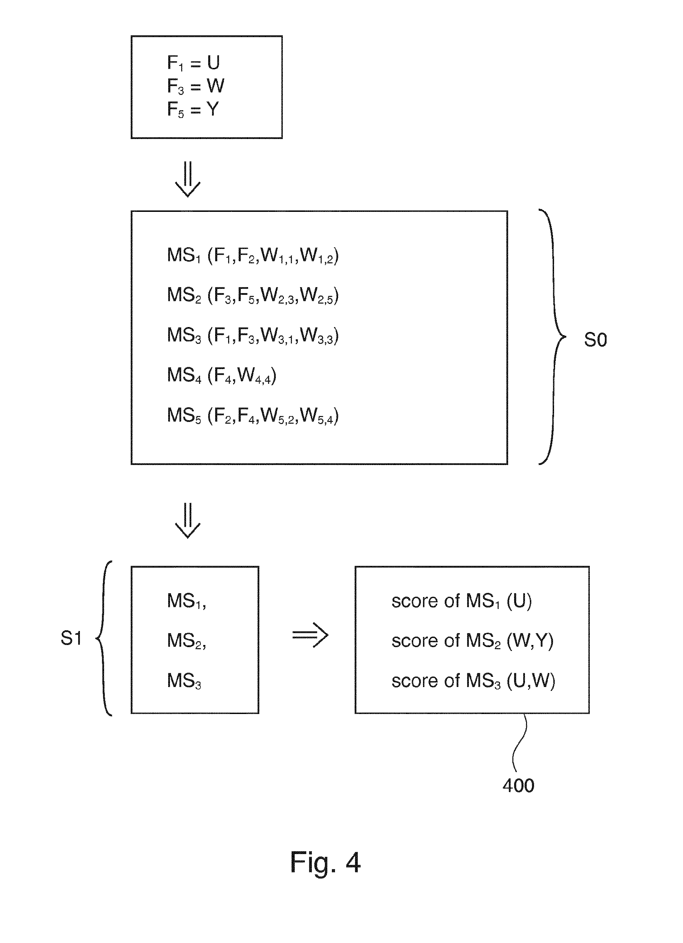

The holistic approach of the herein presented method is reflected in the set-based calculation. If desired, the selection of the subset S1 is carried out such that each medical suggestion comprised by the basic set S0 is evaluated for the selection of the subset S1. In other words, each medical suggestion MSᵢ out of basic set S0 is assessed with respect to the question whether there is at least one association with at least one of the received known facts. This aspect can clearly be gathered from the description of the non-limiting example of Fig. 4. Moreover, if desired, also the calculation of the individual score of the selected medical suggestions MSᵢ can be applied to all MSᵢ of the automatically selected subset S1. In other words, if desired, each selected medical suggestion MSᵢ out of the subset S1 is evaluated during the calculation process.

The currently available knowledge of a medical area can be consolidated by experts and by citing of, for example, guidelines and Standard Operating Procedures (SOP). Such a consolidated medical knowledge can then be digitized according to the structure of the database as described herein and as given in the independent claims. If desired, a sequential, stepwise approach for generating the database may be chosen. In a first step, the knowledge can be "theoretically" integrated into the database by means of the described structure encompassing the medical suggestions MSᵢ, the association with the medical facts Fⱼ, the association with the respective weight W_{i,j} and the exact value distribution of W_{i,j}. Also knock out criteria and/or must have criteria, as defined herein, may be applied. In a second step, a realistic case or event or a plurality thereof is calculated by the system and the presented method. The result of the method is presented to the expert for discussing the outcome and result of the method. Hence, the practical knowledge of the experts can be used to verify results of the method and an adaption of the database as described before is facilitated. This may be seen as a second iteration of generating the database of the present invention in form of feedback of the experts. Consequently, the improved and reliable database for use in the herein presented method and system is generated. A third step may be used to further improve the generated database. In particular, the end user may be provided with a user interface comprising a feedback mechanism which allows, in an individual case, report the "true medical suggestion", for example the true medication. This facilitates a selective deletion of one or a plurality of the presented medical suggestions MSᵢ out of the subset S1 by the user. Based on this behaviour of the user, the system and the method may calculate an adaption of the weights W_{i,j}. Therefore, the feedback of the user for generating and updating the database is provided. In particular, specific problematic scenario events of the medical occurrences can be taken into account for the structure of the database using medical facts Fⱼ of a medical suggestion MSᵢ with weights W_{i,j}.

If desired, the presented methods can make use of a system of "classified probabilities". As an exemplary embodiment, three classes may be used to evaluate the calculated score which may be seen as a probability. The class of suspicion, expressed by a score value between 200 and smaller than 600, the class of a probable assumption, with a score value larger or equal than 600 and below 950, and the class of highly probable assumption with a score value equal to or above 950 points. The herein described scores for point values are always based on an individual case of consideration and evaluation, and is not based on population averaged evaluations. For example, for the individual case the statistically correct statement that 48 % of men older than 80 years suffer from an ischemic heart disease has no value. Either the actually examined 85 year old man suffers from such a disease or does not suffer from said disease. It is impossible to suffer from that disease by an amount of 48 %. In other words, such statistical statements are only valuable if a group of persons is considered. Among 100 men above 80 years, approximately 48 of them suffer from said heart disease. However, this does not help any further for the individual case, when the individual case needs to be examined and analysed in detail. As will become apparent from and elucidated with explanations of the invention, the present invention makes use of such an individual case evaluation, although classified probabilities are used. This will become clear from the following explanations.

Furthermore, the method and/or the system of the present invention is also configured to consider other aspects beside the medical requirements which shall optimize daily diagnostic and therapeutic processes, e.g. economic or administrative requirements. If, for example, a special drug agent is most probably helpful and therefore the best therapeutic option for a patient the method and/or system is able to recommend in addition a special pharmaceutical product (e.g. a specific antibiotic from a certain pharmaceutical company) if the health insurer of the patient runs a 'drug providing contract' with this company. That information can be combined with the medical suggestions provided by the present invention to the user.

Another example for a process optimization is the created score for the 'reliability of the given information' depending on the source/creator of its origin. If an information, e.g. the diagnosis of a mild insufficiency of the Aortic valve, is placed by a general practitioner, this input can be classified the method and/or the system of the present invention as 'probably not as valid' as the same information given by an cardiologist. If the same general practitioner puts into the system of the present invention "use of 100mg ASS per day" to protect the patient from heart problems this knowledge can be ranked higher than the same input from a patient. This "information discrimination" can be important to avoid misleading directions whenever possible. Consequently, the method and/or the system of the present invention can be configured to receive and to process the received known facts in form of values of the medical facts in a format which comprises data about the source/creator of its origin to provide for said information discrimination.

Moreover, he method and/or the system of the present invention may be configured to select different medical suggestions out of the subset S1 based on the medical environment where a decision has to be made. If a patient e.g. visits a GP surgery because of a loss of physical energy and a cardiac murmur is auscultated the system/method would recommend to referral this patient to a cardiologist - because an general practitioner is not able to perform an ultrasound (Echo) examination to identify the cause of the cardiac murmur. If the same patient would consulate a cardiologist the system would recommend as a 'next step' exactly this: an Echo examination which is a standard diagnosis procedure in cardiologic surgeries. Consequently, the method and/or the system of the present invention can be configured to receive and to process the received known facts in form of values of the medical facts in a format which comprises data about the medical environment where the actual decision has to be made.

Another aspect is the geographic localisation which may trigger, i.e. select, different medical suggestions out of the subset S1. For example, a young woman which is found collapsed in Australia with a small wound could be bit by a dangerous snake much more probably than in Norway. Consequently, the method and/or the system of the present invention can be configured to receive and to process the received known facts in form of values of the medical facts in a format which comprises data about the geographical position or geographical origin some or all of the received known facts.

According to another exemplary embodiment of the invention a method of supporting a process of medical decision making in gynaecological endocrinology is presented.

According to another exemplary embodiment of the invention, the subset S1 is characterized in that the medical suggestions MSᵢ comprised in S 1 are each associated with at least one medical fact for which a value was received as known fact.

In other words, only those medical suggestions MSᵢ are selected from the basic set S0 upon receipt of the known facts which medical suggestions, according to the database, comprise an association with received the medical facts Fⱼ. Therefore, medical suggestions are selected by the presented method which does have at least one correlation or link dependency to the medical fact, which values have been received as known facts. In other words, the subset S1 is characterized in that the medical suggestions comprised in S1 are associated with medical facts, values of which medical facts were received as known facts.

According to another exemplary embodiment of the invention, the method comprises the step of assessing each medical suggestion MSᵢ of the basic set S0 upon the step of selecting the subset S1.

In other words, a holistic and entire approach generating a medical suggestion which is useful for supporting a process of medical decision making is presented. According to another exemplary embodiment of the invention, the step of selecting the subset S1 is processed on a set basis.

In other words, the step of selecting the subset S1 is carried out in a set-oriented manner. Moreover, the database of this exemplary embodiment is a relational database which provides certain and distinct advantages compared the hierarchical databases. In particular, in case complex associations or complex relations are comprised or stored in the database, and in case large data volumes are involved, the presented method provides for a fast operation. Further advantages and effects of the set based processes of the present invention are described with respect to e.g. Figs. 2 to 5.

According to another exemplary embodiment of the invention, the medical suggestions MSᵢ are respectively embodied as an element chosen from the group comprising a medical diagnosis, a medical finding, a medication, an anamnesis, an auxiliary suggestion, an evaluation of a lab value, a medical plausibility, a medical conclusion, a medical measure, a medical instruction, a medical statement, a medical question, symptoms, a cluster of symptoms, a text block, a nutrition suggestion, a fitness suggestion, a care suggestion, a rehab suggestion, a genetic aspect, a histology finding, a physiological process, a finding out of a patho-physiological process, a quality indicator, a treatment recommendation, a therapy recommendation, a process suggestion, a medical investigation suggestion, a patient questionnaire, a professional questionnaire, and any combination thereof.

Exemplary embodiments with a detailed and practical description can in addition be gathered from the hereinafter presented examples 1 to 5.

According to another exemplary embodiment of the invention, each respective score of the medical suggestions MSᵢ of the subset S1 represents a probability that the respective medical suggestion is correct.

Such calculated probabilities may be sorted or organized in a list and this list may be provided to the user. Specific categories of probabilities like suspicion, probable and highly probable can be used in order to classify the calculated scores. If desired, only a selection of such categories may be displayed or output to the user.

According to another exemplary embodiment of the invention, the medical facts Fⱼ are respectively embodied as an element chosen from the group comprising an age of the patient, a gender of the patient, a body weight of the patient, a body height of the patient, a physiological parameter, a biological parameter, a chemical parameter, a medical parameter, a symptom, an information associated with a medical complaint, a result of a medical finding, information associated with living conditions of the patient, information about the patient which is useful for describing a medical situation of the patient, a diagnosis, medical data, medication data, fitness data, nutrition data, rehab data, care data, telemetry data, statistical data, medical reference data, an anamnesis, a risk factor, an allergy, a habitat of the patient, a job situation of the patient, a housing situation of the patient, imaging data, regional weather data, regional environmental data, endemic data, epidemic data, a result of a function test, information received from a professional or the patient via a questionnaire and any combination thereof.

Furthermore, individual circumstances of the life of the individual patient could be used as medical fact. For example, stress or pressure in personal relationships or in the context of the working circumstances, expositions to polluting or environmentally harmful circumstances or hobbies may be alternative embodiments of medical facts. Regarding the medical fact "regional environmental data" it should be noted that for example data like regional radiation exposure, environmental pollution, and the presence of substances like lead, ozone, ultraviolet radiation, fine dust, or noise may be embodiments thereof.

According to another exemplary embodiment of the invention, the step of calculating the score of medical suggestions comprises the steps weighting a received first value of a first medical fact Fⱼ of a first medical suggestion MSᵢ by applying a first weight W_{i,j} resulting in a first suggestion result. Further, the step of weighting a received second value of a second medical fact Fₖ of the first medical suggestion MSᵢ by applying a second weight W_{i,k} resulting in a second suggestion result is comprised. Moreover, the step of summing the first suggestion result and the second suggestion result to the score of the suggestion Sᵢ is carried out by the presented embodiment of the invention.

In other words, in this case the score of the medical suggestion MSᵢ can be seen as a sum of the weighted individual result. Also aspects of the previously described specificity selection may used in addition or alternatively for calculating the score of a medical suggestion.

According to another exemplary embodiment of the invention, the method further comprises the step of weighting a combination of the first medical fact Fⱼ and the second medical fact Fₖ by applying a combination weight Wᵢ, combination ⱼ₋ₖ, and wherein the combination is chosen from the group of Boolean combinations comprising AND, OR, AND NOT, and any bracket combination thereof.

In other words, the provided database comprises such used weights for the combinations of different medical facts Fⱼ and Fₖ. Moreover, the database, which can be a relational database, comprises the applied combinations out of the group of Boolean combinations. In other words, this embodiment of the present invention takes into account not only the single set or occurrence of the first medical fact Fⱼ and the second medical fact Fₖ, but weighs that both facts are received and also weighs the values of Fⱼ and Fₖ. Consequently, this method during the step of calculating the respective score takes into account the relations of the medical facts Fⱼ and Fₖ. Explicit examples will be given later on.

According to another exemplary embodiment of the invention, the method comprises the step of repeating previously described steps with respect to at least one second medical suggestion Sₘ.

Of course, the presented method may repeat said steps also for a large plurality of medical suggestions which are comprised within the selected subset S1. Only the medical suggestions which are attributed with a comparatively high score can be displayed to the user as probable, highly probability if desired.

According to another exemplary embodiment of the invention, the weight W_{i,j} is a probability distribution which expresses the probability that the corresponding medical suggestion MSᵢ is correct, based on a value of the medical fact Fⱼ.

According to another exemplary embodiment of the invention, the step of calculating the score of medical suggestions comprises the steps receiving a first value of a first medical fact Fⱼ of a first medical suggestion Sᵢ, receiving a second value of a second medical fact Fₖ of the first medical suggestion Sᵢ, and weighting a combination of the first medical fact Fⱼ and the second medical fact Fₖ by applying a combination weight Wᵢ, combination ⱼ₋ₖ.

The combination weight may be embodied in various different ways. For example, the weight Wᵢ, _{combination j-k}.is embodies as a probability distribution which expresses the probability that the corresponding medical suggestion MSᵢ is correct, based on the combination of values of the medical facts Fⱼ and Fₖ. In other words, taking into account the specific values of the medical fact Fⱼ and Fₖ, which values have been received by the system, the holistic and entire approach of the present invention based on a set oriented calculation method, is provided.

According to another exemplary embodiment of the invention, the database provides for a structure such that all medical facts Fⱼ are autarkic and equivalent.

In other words, the none medical fact is preferred with respect to another medical fact, no priority is applied to any medical fact. Therein, the aspect of the medical reality is reflected, that medical interrelations and dependencies occurs in a net-like manner, and not in a tree-like manner. In other words, no hierarchy of the medical facts Fⱼ is comprised in the database.

In another embodiment, gender and or age of the patient may be considered as exceptional medical facts in the sense of general data which are regarded more important than other facts due to their generality.

According to another exemplary embodiment of the invention, the method comprises the steps ranking the medical suggestions MSᵢ of the subset S1 in an order of their respective calculated score, and providing the order of ranked medical suggestions to the user.

If desired, only those medical suggestions MSᵢ may be presented and provided user which exceed a specific predetermined threshold. Such a threshold may be adapted and individually amended by the user.

According to another exemplary embodiment of the invention, the method comprises the steps of supplementing the received known facts by at least one medical suggestion MSᵢ out of the subset S1 based on the respective calculated score of said at least one medical suggestion. The method further comprises the step repeating steps 2 to 4 as described previously with the supplemented received known facts.

In other words, the method facilitates that a result of the first iterative calculation or a part of said result can be used as an input for the second or further iteration of the herein presented method. In other words, the method is able to produce and generate new, additional facts based on those medical suggestions MSᵢ of the first iteration, which are calculated as being the corresponding score. The threshold may be used to determine whether the score is high enough. In case the score of the medical suggestion exceeds said threshold, the method automatically provides this medical suggestion as a known fact Fⱼ for the next process which again carries out steps 2 to 4. Consequently, this exemplary embodiment facilitates a user controllable and user selectable supplementation of the input data, i.e. of the received known facts for the second or for further iterations.

According to another exemplary embodiment of the invention, the method further comprises the steps of providing for a score threshold, and selecting the at least one medical suggestion out of the subset S1 if the score of said medical suggestion is larger than the score threshold.

As has been described in the context of the previous exemplary embodiment, medical suggestions within appropriate score can be transformed by the herein presented method into medical facts for the subsequent and following iteration of the method. Therefore, the method may be seen as an iterative method. The score threshold, as well as other thresholds used herein, may be provided in the database. Also other criteria, depending on the definition of the score may be chosen. For example, larger or equal than the score threshold, smaller than the score threshold, equal to the score threshold are possibilities of embodiments.

According to another exemplary embodiment of the invention, the method further comprises the steps of classifying the individual received known facts with respect to the respective creator of said received known facts, and applying a prioritization of the received known facts based on the respective creator.

In particular in the context of the previously described embodiments regarding the transformation of a medical suggestion into a medical fact for further iterations, its classification and prioritization may be of importance. The creator of said received known facts may also be seen as the source of said received known facts. Advantageously, this embodiment facilitates the distinction between received known facts from the "true world" and may be termed "real medical facts" whereas the transformed medical suggestions, which subsequently are used as medical facts, can be classified as "virtual medical facts". In other words, this exemplary embodiment of the invention facilitates a distinction between medical facts regarding their quality or regarding their origin. Exemplary creators of received known facts may be the patient, a general practitioner, a specialized medical doctor, a customer device or a device of the medical doctor. Corresponding prioritization of the respective known facts can be carried out and applied by the present invention. This further increases the reliability and accuracy of the presented method.

In particular, the method and system of the present invention are configured to take into account the creator or source of the received medical facts or of information which is provided to the database. The fact whether a value of a medical fact Fⱼ originates from a patient, a general practitioner, a medical specialist, a consumer device or a professional device can be decisive with respect to the output generated by the present invention for the user. In particular, different weights may be applied by the method or the system, depending on the creator of the corresponding medical fact Fⱼ, i.e. the source of the medical fact Fⱼ. Moreover, in case the calculated scores presented to the user, the profile of the individual user can be taken into account by the method and the corresponding system. For example, examination methods may be selected out of the subset S1, which methods can be preferred by the individual user currently using the presented invention. In case the individual user has an X-ray or computer tomography device at his disposal, the method and system of the present invention may prefer a corresponding medical suggestion as an output or may increase the score correspondingly. Moreover, the user can interactively provide information to the system carrying out the present invention regarding the calculation profile he wants to apply. For example, as a first profile "calculation of a result as soon as possible" can be chosen, alternatively the profile "cost effective target orientation", or alternatively "legally compliant procedure" can be chosen by the user. However, also other profiles are imaginable. The method and the corresponding system can be configured to provide such profiles to the user for selection via for example a user interface.

According to another exemplary embodiment of the invention, at least one of the received medical facts Fⱼ is provided in form of a time evolvement.

Generally, different forms of time evolvements may be used. For example, a matrix with time-dependent values of blood pressure or heart beats per minute may be an embodiment. Furthermore, a diagram of a time evolvement of a specific physiological parameter may be another embodiment. Said diagram of a time evolvement may be provided in form of mathematical function of description like e.g. a trend function.

According to another exemplary embodiment of the invention, said time evolvement is represented by a vector comprising n values of the medical fact at n different points in time.

For example, n=4 for the medical fact Fⱼ embodied as the body weight, the corresponding vector of the medical fact may be provided in the following form: [60 kg (at 15.12.2010) 70 (at 27.2.2010), 75 kg (at 31.12.2010), 73 kg (at 5.1.2011)].

According to another exemplary embodiment of the invention, at least one of the received medical facts is provided in form of longitudinal patient data. In other words, patient data are provided in a time sequence, which can be embodied in various different ways. For example an average value may be presented, for example an average value of the last three months. Also a trend function may be used, indicating an increasing tendency or a decreasing tendency within a specific period, like for example half a year. Furthermore, existent functions can be used. Moreover, the fluctuation margin may be used as a medical fact or also a minimum function or a maximum function may be used such that statement "in the time period x the value has a minimum of y". Also a sum function may be used as such a longitudinal patient data providing the information that "the sum of the occurrences, e.g. hospital admissions per month, exceeds a threshold x within the last year" is possible.

According to another exemplary embodiment of the invention, the basic set S0 comprises a plurality of medical suggestions which are respectively embodied as diagnoses. Furthermore, each diagnosis is associated with medical facts which are embodied as a symptom or as a medical fact which is relevant for or is associated with said diagnosis. This exemplary embodiment of the present invention will be become apparent from and more elucidated with the description of the following examples 1 to 5.

According to another exemplary embodiment of the invention, a first medical suggestion of the basic set S0 is associated with a second medical suggestion such that during the step of calculating the score of the first medical suggestion, a score of the second medical suggestion is calculated.

In the following, a corresponding situation is exemplarily described. Hyperandrogenaemia is defined by increased androgens, like testosterone or DHEAS, in the blood of the patient. Androgens themselves depend from medical facts like gender, age, state of pregnancy and may be from the measurement method used by the lab. Whether an androgene value is above the reference region can be evaluated in a corresponding medical suggestion by the present invention. The result of said medical suggestion in turn influences the medical suggestion "diagnoses hyperandrogenaemia". In addition, the androgen values, besides hyperandrogenaemia, influences other medical suggestions. An important advantage of this exemplary embodiment may be seen in the following. In case a further androgen-dependent medical suggestions is found or in case the applied measurement methods are adapted or become more precise, only the corresponding weights have to adapted. Advantageously, not all medical suggestions which comprise an association with the androgen value, like e.g. hyperandrogenaemia, have to be adapted in the database. Also hyperandrogenaemia is a medical suggestion which may play a role in other medical suggestions. For the medical experts which create the database the structure of associations, dependencies and correlation is provided in a manageable structure or format.

According to another exemplary embodiment of the invention, at least one medical suggestion of the basic set S0 is associated with a plurality of medical facts Fⱼ, and wherein at least a part of said plurality of medical facts are thematically linked together in the database to form a group of medical facts.

The use of formed groups of medical facts facilitates the delimitation of the set towards a topically linked subset of medical suggestions. Such a group of medical facts may represent a composition of medical facts regarding a specific medical aspect. From a structural point of view, such a group may be seen as one structure above the medical facts and one structure below the medical suggestion. In particular, such a group may comprise several medical facts, whereas a medical suggestion may comprise or may be associated with the selection of medical facts and a group of medical facts. For example, such a group may be "symptoms of hypothyreosis" and may comprise the following medical facts:
● increase of body weight: yes/no/unknown,
● general physical weakness: yes/no/unknown,
● loss of appetite: yes/no/unknown,
● listlessness: yes/no/unknown,
● dry skin: yes/no/unknown,
● constipation: yes/no/unknown,
● hair loss: yes/no/unknown,
● and in case of women absence of bleeding: yes/no/unknown.

The corresponding medical suggestion may be "diagnosis of hypothyreosis" in which the presented group of medical facts can be effectively used. Moreover, said exemplary group of medical facts can easily be used in other medical suggestions, such that in case of an amendment or an adaption of the group, as associated medical suggestions are updated centrally and at once. Simultaneously, all medical suggestions using said group are updated and/or amended automatically.

According to another exemplary embodiment of the invention, said database comprises at least one medical suggestion MSᵢ which is associated with a knockout criterion for said medical suggestion.

In other words, in case one received known fact in form of a value of a medical fact presupposes that the medical suggestion MSᵢ cannot be true, the respective medical suggestion is removed from the used subset S1 or is not selected at all. In case the medical suggestion is exemplarily embodied as "pregnancy yes or no", the gender of the patient is a so-called knockout criterion. In case the received known facts comprise that the gender of the patient is male, the medical suggestion "pregnancy yes or no" is removed from the used subset S1 or is associated with a remark that it is not used any more for further calculations.

According to another exemplary embodiment of the invention, said at least one medical suggestion is not selected for the subset S1 in case the received known facts fulfil said knockout criterion.

According to another exemplary embodiment of the invention, at least one medical suggestion MSᵢ is associated with a must have criterion for said medical suggestion in the database.

In other words, in case a required medical fact or a required value of a medical fact is not received as the input for processing the method, the corresponding medical suggestion MSᵢ associated with the so-called must have criterion is not selected for the subset S1.

According to another exemplary embodiment of the invention, the at least one medical suggestion is only selected for the subset S 1 if the received known facts fulfil said must have criterion.

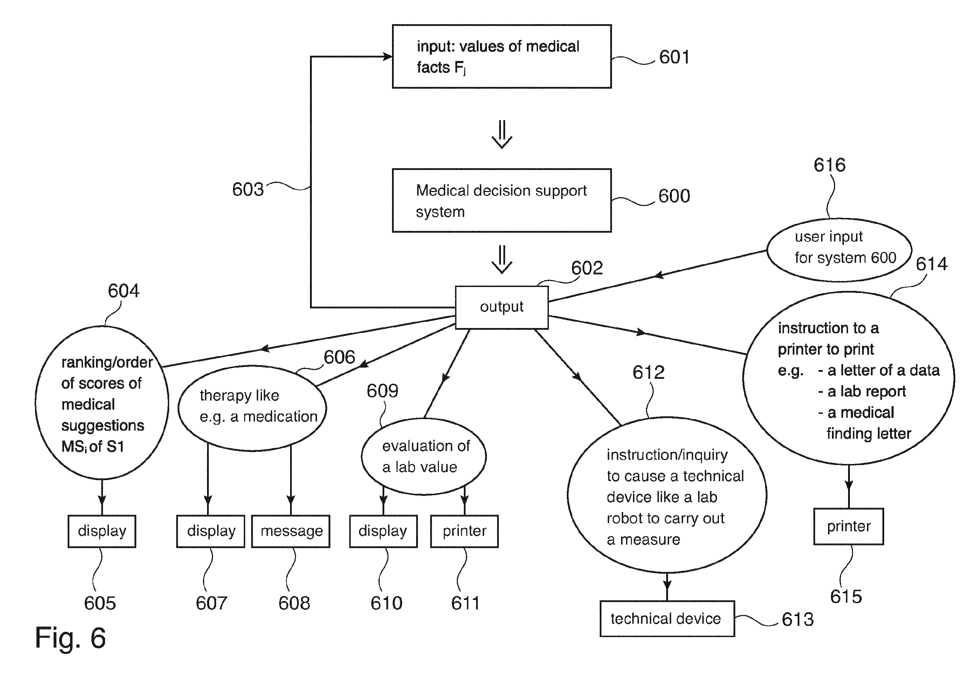

According to another exemplary embodiment of the invention, the method further comprises the step of generating an output based on the calculated scores, wherein the output is chosen from the group comprising a list of probable medical suggestions ranked in the order of the respective calculated score, a report, a letter addressed to the patient, a letter addressed to lab, a letter addressed to a lab comprising an instruction or suggestion for a further measurement in said lab, a medical finding letter, a medical finding letter with a sender identification of a clinician, a question or a question set to a user, a question to a user in form of a graphical interface, an order, an order of a medicament, and any combination thereof. Fig. 6 depicts such context schematically.

According to another exemplary embodiment of the invention, the method further comprises the step of providing said generated output to the user in form of output data, receiving amendment information about an amendment of the output data caused by the user, and adapting said database based on the received amendment information.

In the following self-learning aspects of the system and the method are described. The herein presented method and system are configured to learn from the behaviour of the user in a passive and automatic way and may use information about the user behaviour to update the database in view of selections of suggested MSᵢ carried out by the user. In particular, if a plurality of medical suggestions MSᵢ are presented to the user as a result of the method and the user selects one of said suggestions, this user selection may be provided as a feedback information to the system or the database, such that an adaption of the corresponding associations of said medical fact and/or an adaption of the corresponding weight W_{i,j} can be carried out. Moreover, feedback system is provided by the method and the system of the present invention. In case a wrong decision or a wrong medical suggestion is presented by the method or the system, the user is provided with the possibility, e.g. a user interface button, to let the system know that a wrong decision was provided.

Moreover, the method and the system are configured and facilitated receiving or inquiring information from external technical devices about the final, "true suggestion" or "true diagnosis" such that a subsequent adaption of the database is facilitated.

According to another exemplary embodiment of the invention, the adaption of the database is chosen from the group comprising of adapting an association of at least one medical suggestions with at least one respective medical fact Fⱼ, adapting at least one weight W_{i,j}, adapting selection rules for selecting the subset S1 of medical suggestions out of the basic set S0, and any combination thereof.

According to another exemplary embodiment of the invention, the steps of selecting the subset and of calculating the scores is carried out by the calculation unit, the method further comprising the step providing for an interface between the calculation unit and a medical fact source for facilitating data transmission between the database and the medical fact source. Therein, the interface is configured to facilitate transmission of known facts in the form of values of medical facts Fⱼ of at least one individual patient to the calculation unit when the medical fact source is connected to the interface.

In the following the use of external data by the present invention is described. The method and the system of the present invention are configured for and facilitate the use of external data, and allow an integration of said data into the database. Preferably, external structured data may used. The presented method and system allow for a data transfer as international structures like the International Statistical Classification of Diseases and Related Health Problems (ICD) for diagnosis, the Anatomical Therapeutic Chemical (ATC) Classification system for medicaments, the Pharma Zentral Nummer (PZN), Health Level Seven (HL7), the format xDT, or Systematized Nomenclature of Medicine Clinical Terms (SNOMED CT) and others can be used. Consequently, the herein presented method and system are configuration to access structured medical knowledge and make use of said knowledge during or for the generation of the medical suggestion described herein.

According to another exemplary embodiment of the invention, the calculation unit is configured to process said known facts received from the medical fact source. Therein, the medical fact source is chosen from the group comprising a sensor, a sensor for determining a physiological parameter, a smart phone with sensor or data, a data system of a medical practice, a data system of a hospital, a data system of a care or rehab organization, a network of a medical office and/or hospitals/a care or rehab organization or a network of integrated care, a database of a medical office, a database of a hospital/care or rehab organization, a database of national medical networks , a software providing structured medical, environmental or statistical data, a lab device, and a lab robot.

According to another exemplary embodiment of the invention, the method is carried out via a computer network. According to another exemplary embodiment of the invention, the steps of selecting the subset S1 and calculating the scores is carried out on a server or on a server cluster. According to another exemplary embodiment of the invention, the step of receiving known facts is carried out via a user interface by means of which the user enters the values.

According to another exemplary embodiment of the invention a program element is provided and according to yet another exemplary embodiment of the invention a computer readable medium is provided.

The calculation unit may be embodied as a processor or a PCU but may also be embodied differently. The program element may be part of a computer program, but it can also be an entire program itself. For example, the computer program element may be used to update an already existing computer program to get to the present invention. The computer-readable medium may be a storage medium, such as for example a USB stick, a CD, a DVD, a data storage device, a hard disc, or any other medium, in which a program element as described above can be stored. These and other features of the invention will become apparent from and be elucidated with reference to the embodiments described hereinafter.

According to another exemplary embodiment of the invention, a medical decision support system for generating a medical suggestion useful for supporting a process of medical decision making is presented. The system comprises a database with a basic set S0 of medical suggestions MSᵢ, wherein in the database at least some of the medical suggestions are associated with at least one respective medical fact Fⱼ. Furthermore, in the database the respective medical fact Fⱼ of a medical suggestion MSᵢ is associated with a weight W_{i,j}. The system further comprises a received apparatus for receiving known facts in the form of values of medical facts Fⱼ, which known facts are associated with an individual patient. The system further comprises a calculating unit configured for selecting a subset S1 of medical suggestions MSᵢ out of the basic set S0 based on the received known facts. Furthermore, the calculation unit is configured for calculating a respective score for at least some medical suggestions MSᵢ of the subset S1 based on the received values of the medical facts Fⱼ and the respective weight W_{i,j}.

As has been described before, this medical decision support system can be configured to carry out each of the herein presented embodiments relating to the method.

### BRIEF DESCRIPTION OF THE DRAWINGS

Fig. 1 illustrates a flow diagram of a method according to an exemplary embodiment of the invention.
Fig. 2 exemplarily shows a set based generation of a medical suggestion which can be used in a method according to an exemplary embodiment of the invention.
Fig. 3 schematically shows an iterative set based generation of a medical suggestion which can be used in a method according to an exemplary embodiment of the invention.
Fig. 4 schematically shows a method of generating a medical suggestion according to another exemplary embodiment of the invention.
Fig. 5 schematically illustrates a comparison between tree diagram based systems, i.e. prior art, and a set oriented method of generating a medical suggestion used in an exemplary embodiment of the invention.
Fig. 6 schematically shows a medical decision support system according to an exemplary embodiment of the invention.
Fig. 7 schematically shows medical decision support system according to another exemplary embodiment of the invention.

### DETAILED DESCRIPTION OF EMBODIMENTS

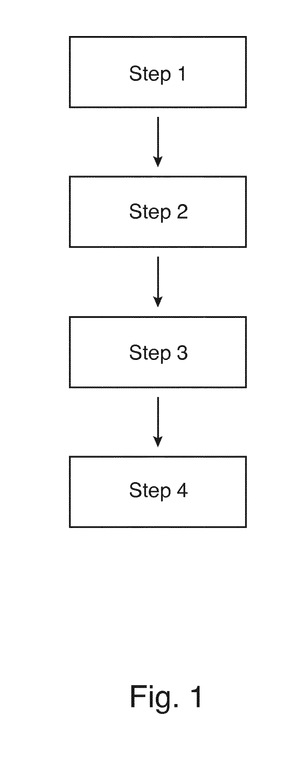

In Fig. 1 shows flow diagram of a method of generating a medical suggestion MSᵢ is presented. Said medical suggestion MSᵢ can be used by the medical doctor, the patient, or another user in the medical context as a support for the process of medical decision making. In step 1 of this method, a database with a basic set S0 comprising a plurality of medical suggestions MSᵢ is provided. The database can be embodied as a relational database. Moreover, in that database, at least one, some, or all of the medical suggestions MSᵢ comprised by the basic set S0 are associated with at least one respective medical fact Fⱼ. In a further practical application, each medical suggestion MSᵢ is associated with a plurality of different medical facts Fⱼ. Furthermore, in the database of Fig. 1 the at least one respective medical fact Fⱼ of the at least some medical suggestions MSᵢ is associated with a weight W_{i,j}. In step 2, known facts in the form of values of medical facts Fⱼ are received, wherein the known facts are associated with an individual patient. During the method step 3 a selection of medical suggestions MSᵢ out of the basic set S0 based on the received known facts is carried out, which leads to a selected subset S1. In addition, step 4 which represents the calculation of a respective score for at least some medical suggestions MSᵢ of the subset S1 based on the received values of the medical facts Fⱼ and the respective weight W_{i,j}. If desired, different method steps described herein can be supplemented like the generation of new medical facts. The method may also be supplemented to provide for an iterative method which carries out the steps several times.

In a further practical embodiment, the respective score for each medical suggestion MSᵢ out of the selected subset S1 is calculated. In other words, an automatic method is presented in which an input in form of known medical facts is received, in which a calculation regarding scores of potential medical suggestions MSᵢ are calculated and which may comprise an output provided to the user in form of scores of the medical suggestion which have previously been selected. If desired, the method depicted in Fig. 1 can be supplemented by the user according to the previously described embodiments of the present invention and by embodiments which will be explained hereinafter.

Fig. 2 schematically shows how an exemplary embodiment of the method of Fig. 1 may carry out the method of generating a medical suggestion MSᵢ. In particular, a set based generation of a medical suggestion MSᵢ is depicted in Fig. 2. Starting with a basic set S0, which is stored in a database 201, a physical entity like for example calculation unit 200 may calculate the subset S1 based on the received known facts Fⱼ. As can be seen from Fig. 2, S1 is a subset of the basic set S0. The principle of the process of restricting the basic set S0 to the subset S1 and the criteria used by the presented method for that restriction/selection can also be gathered from Fig. 4. In the example of Fig. 2, only the medical suggestions MS₃, MS₄, and MS₆ do fulfil the subset criterion of S1. Said criterion used for the selection of subset S1 may be embodied in different ways. Exemplary embodiments of such criterion are described hereinafter and have been described already before. The example of Fig. 2 also calculates a respective score of the selected medical suggestions MS₃, MS₄, and MS₆ based on the associations of those medical suggestions with the received known facts F₁, F₂, and F₃ according to the content of the database 201. The calculation unit 200 can also be implemented in a network system. Moreover, a server or a cluster of server may be used to carry out said method. The received known facts 202 are as follows. F₁ is the medical fact blood pressure and a value of this medical fact received is 110/70. This is a known fact associated with an individual patient. The medical fact F₂ is embodied as pulse, the value of which is 95 beats per minute as a value of an individual patient. Moreover, the third received known fact F3 is embodied as body temperature of the patient and is provided as a value of 40 °C. The known facts are received by the calculation unit 200, which acts on the database 201 in which the decisive associations and weights W_{i,j} are stored. The basic set S0 shown with reference sign 203 in this embodiment is also stored on the database 201 and is depicted in Fig. 2 as an enlarged view. The subset S1 is depicted as 204. Moreover, the example of Fig. 2 comprises a calculated subset S2 with medical suggestions MS₄, having a calculated score which is larger or equal than a score threshold x. Consequently, the result of the method depicted in Fig. 2 is the medical suggestion MS₄ which can be embodied for example as a diagnosis, as a question provided to the user, as a letter directed to a lab for enquiring a further analysis of for example a sample probe of the patient.

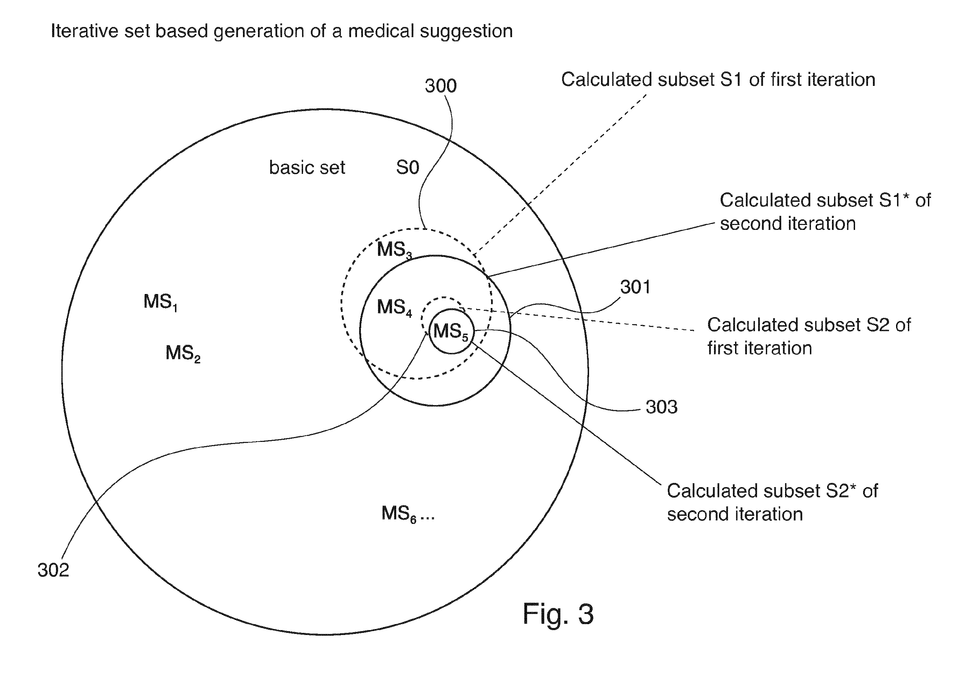

The embodiment shown in Fig. 3 may be seen as a further development of the embodiment of Fig. 1 and/or of Fig. 2. In particular, an iterative set based generation of a medical suggestion MSᵢ is illustrated in Fig. 3. As a result, the medical suggestion MS₅ is presented to the user. Regarding the structure of the received known facts, the calculation unit, and the database, it is kindly referred to Fig. 2. However, also other IT structures as described herein may be used for the embodiment of Fig. 3. In addition to the calculation of the subset S1 in a first iteration shown with reference sign 300 a second iteration of the method steps described with respect to Fig. 1 can be carried out in the embodiment of Fig. 3. The result is depicted as calculated subset S1* 301 of the second iteration. The calculated subset S2, shown as 205 in Fig. 2, corresponds to the calculated subset S2 shown with reference sign 302 in Fig. 3. Additionally, Fig. 3 comprises the calculated subset S2* of the second iteration which results in the subset 303 comprising the medical suggestion MS₅. Due to the further iterations, i.e. the multiple and/or repeated processing of at least a part or all of steps 1 to 4 of Fig. 1, an increased accuracy and an improved reliability of the correctness of the result MS₅ is achieved by the embodiment of Fig. 3. In particular, the way of finding the result MS₅ is based on pure set based limitation of the basic set S0 via subset S1, subset S1*, subset S2, and finally subset S2*.

According to another exemplary embodiment of the invention, Fig. 4 shows a method of generating a plurality of medical suggestions useful for supporting a process of medical decision making. In particular, the result 400 is embodied as three different scores of the medical suggestions MS₁, MS₂, and MS₃, respectively. Depending on the received known facts F₁, F₃, and F₅, in form of the value U, W, and Y, the basic set S0 is evaluated and a selection of the subset S1 is conducted. Only medical suggestions MSᵢ are selected for the subset S1 which comprise an association with at least one medical fact Fⱼ in the database for which values have been received as known facts. In Fig. 4 the dependencies of the used medical suggestions MS₁ to MS₅ from the medical facts Fⱼ to F₅ and the correspondingly used weight W_{1,1} to W_{5,5} are explained in detail. However, the present invention is not limited to such a numerical example and can be extended to a large plurality of medical suggestions, each depending on a plurality of corresponding medical facts. In particular, a set based generation of a medical suggestion, which may be seen as a set oriented approach of selecting medical suggestions out of the basic set S0 is an advantageous approach in scenarios where a large database with a large number of medical suggestions have to be evaluated in view of a large number of received known facts. Such scenarios of the present invention due to its set based aspects allows for a reliable efficient medical decision support.

In the example of Fig. 4, values U, W, Y have been received as known facts and are values, characteristics and/or markednesses of the medical facts F₁, F₃, and F₅. Consequently, the calculation unit selects the medical suggestions MS₁, MS₂, and MS₃ out of S0 to define the subset S1. As the remaining medical suggestions MS₄ and MS₅ do not comprise an association with at least one of the medical facts F₁, F₃, and F₅, they are not selected for the subset S1.

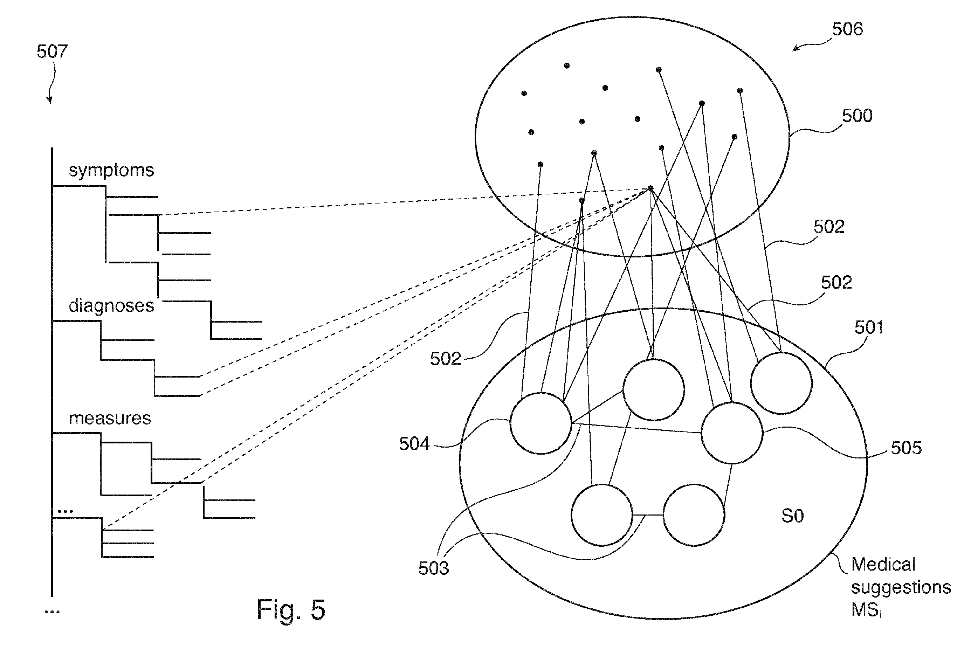

Fig. 5 schematically shows advantages of the set based method of generating a medical suggestion, using a relational database comprising the herein described associations between the medical suggestions MSᵢ comprised by basic set 501, shown on the right hand side of Fig. 5, in contrast to a disadvantageous tree based method of generating medical suggestions used by the prior art. The tree based method is shown on the left hand side of Fig. 5. The medical facts Fⱼ used in this exemplary embodiment are depicted in the circle 500. The depicted lines 502 are an illustration of the respective associations. In addition, the corresponding weights W_{i,j} are comprised and defined by the corresponding database. Moreover, lines 503 depict the situation that a first medical suggestion can be associated with a second medical suggestion. For example, medical suggestion 504 can comprise medical suggestion 505, such that during the calculation of the score of medical suggestion 504, the score of the score medical suggestion 505 is calculated. This facilitates a simplified data structure used in the database and may increase the speed of the herein presented method. Consequently, time required for a response to the user is decreased by this exemplary embodiment. The set based structure 506 shown on the right hand side of Fig. 5 is an effective representation of medical reality, which indeed is netlike with respective to the different correlations between medical facts and medical suggestions. In case of complex associations/interrelations and in case of large date volumes, structure 506 provides for a fast operation of the method. Advantageously, the structure 506 can be implemented in a relational database. This provides for specific advantages in contrast to hierarchical structure 507, as used by prior art systems. Structure 506 provides for simplified maintenance, as the single components of structure 506 do not have to be ordered in a strict hierarchical order. Disadvantageously, the prior art structure 507 strictly requires a correct hierarchical order of the respective elements to avoid any inconsistency.

Moreover, the advantageous structure 506 of the present invention allows for a concurrent targeting of different concepts or medical suggestions MSᵢ whereas in the prior art structure 507, a single path through the tree based diagram needs to be selected by the user. However, such a selection entails the risk of ignoring or underestimating one element of the received known facts. Moreover, the structure 506 of the present invention allows for an improved representation of uncertain or inexact knowledge. In contrast thereto, the prior art structure 507 requires a hierarchical relationship. In case one medical fact is used in several different branches of the tree structure 507, the corresponding calculation process slows down in the tree based system, as complicated calculation operations have to be carried out. Moreover, a further advantage of the structure 506 of the present invention is the ability to adapt the used sets like basic set S0 and selected set S1, for example, in view of new requirements or user requests. Such an adaption can be carried out in a highly dynamic manner. Based on each new medical fact, for example in the used input vector, a new set is generated in a dynamic way. Moreover, the use of sets in the context of medical decision support provides for the possible and advantage of an improved delimitation of the used sets. For example, in case of a specific method area, like for example heart surgery, the method or aspects of the method like the calculation of the subset or of the scores can be restricted to only a part of the basic set S0. This may increase the speed of a delivery of a response to the user. In contrast thereto, prior art structure 507 disadvantageously comprises holes within its structure 507 when a delimitation for a specific medical area is required/applied. The advantages described for structure 505 can be applied to any embodiment of the present invention.

According to another exemplary embodiment of the invention, Fig. 6 shows a medical decision support system 600. The system 600 is configured to carry out the methods described with respect to Figs. 1 to 4. As shown in Fig. 6, the system 600 creates an output 602 which can be embodied in various different ways and which can be used in various different ways according to the present invention. For example, the output may be a ranking/an order of scores of medical suggestions MSᵢ of the subset S1 and may be transmitted to a display 605. The output 602 may also be used as a feedback 603 and may be provided in addition to the values of medical facts Fⱼ which were received in a first iteration to carry out a second or a further, like a third or fourth iteration of the method. Furthermore, Fig. 6 shows that the output 602 may be embodied as a suggested therapy like e.g. a medication 606. These medical suggestions may be displayed on display 607 or may be provided to the user by means of a generated message 608. Another possibility of embodying the output 602 is the evaluation of a lab value shown with 609 which evaluation may be sent to a display 610, or may be sent to a printer 611 to automatically create a printed document. The output 602 according to Fig. 6 can also be embodied as an instruction or inquiry 612 to cause a technical device like a lab robot to carry out a measure. These medical suggestions 612 may be sent to a technical device 613, which may be embodies as said lab robot. Additionally, output 602 can be an instruction to a printer to print e.g. a letter of a doctor, a lab report or a medical finding letter. Printer 615 can be automatically provided by the system 600 with such instructions. Moreover, the input of a user 616 may be provided to the system 600 by means of for example an interface of the user. For example, the user may select a plurality of suggested medical suggestions MSᵢ, which are provided with respective scores, for to use the selected medical suggestion or suggestions, the feedback 603 for the input 601 or a second iteration. Consequently, the shown elements 604, 606, 609, 612, 614, and 603 may be seen as medical suggestions MSᵢ that are generated by the herein presented method.

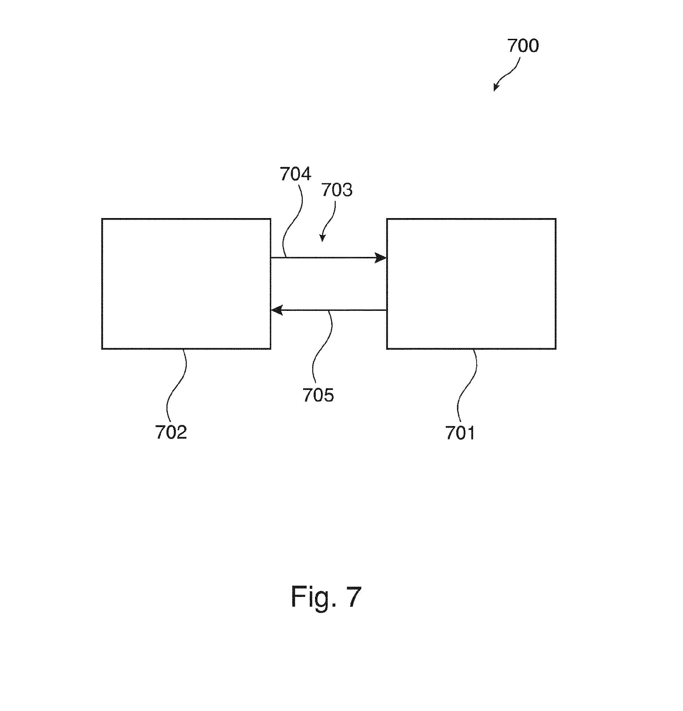

Fig. 7 schematically shows another exemplary embodiment of a medical decision support system 700, which comprises a user interface 702 and a server 701, which communicate with each other by means of network 703. The known facts in form of values of medical facts Fⱼ may be provided by the user interface 702 in form of instructions via communication path 704 to the server 701. Moreover, the server 701 may carry at least one step or all steps shown within Figs. 1 to 4. The server 701 may provide the user interface 702 with calculated scores for the selected medical suggestion MSᵢ and thus provides for respective probabilities that the selected medical suggestions MSᵢ are correct. This result provision may be carried out via communication path 705.

For a complete understanding of the broad application variety of the present invention the following two examples are presented.

### Example 1: Calculation of a lab value

### Target:

Evaluation of a measured lab value with respect to its reference region.

### Background:

In the field of gynaecological endocrinology, lab values are dependent from a large variety of factors. Therefore, a measured value having the numerical value x is within the reference region and thus normal the first day, whereas the same value x is far distant from said reference region and thus highly pathological when occurring at a different day.

The medical situation for e.g. the FSH value and its dependencies are as follows: The reference region of the FSH value depends on the day of cycle and on the age of the patient. Consequently, for evaluating whether a given FSH value is within the reference region, the day of cycle, the age of the patient as well as the current FSH value should to be known. As will become apparent from and elucidated with the first example shown here, the method and system of the present invention facilitate a reliable and efficient calculation of a result for e.g. the FSH value when an input is provided according to known medical facts F₁ to F₉ as exemplified here for Example 1. The present invention provides for an improved method and system to assist the user during such a scenario, making a medical decision.

### Procedure:

In this embodiment, the basic set S0 comprises 300 medical suggestions MS₁ to MS₃₀₀. In particular, the 300 medical suggestion are composed or defined in the database as follows. In this example, one is interested in the use of the 30 most important, commonly used lab parameters of gynaecological endocrinology. Said 30 parameters constitute 30 medical facts F₁ to F₃₀. Each of said parameter is combined in this example with five different medicals facts regarding the individually received value and its relation to the corresponding reference region. Said five different medicals facts, F₃₁-F₃₅, can be summarized as follows for one parameter:
● lab value x is strongly below the reference region.
● lab value x is below the reference region.
● lab value x is within the reference region.
● lab value x is above the reference region.
● lab value x is strongly above the reference region.

This already leads to 150 medical suggestions each comprising a combination of one of the 30 parameters and one of the five "reference region" medical facts. With respect to overview, calculation speed and data maintenance reasons, it might be desirable to further take into account the medical fact "pregnancy" with the possible values "yes" and "no". This again doubles the set of medical suggestions in this scenario to a total amount of 300 medical suggestions MSᵢ.

In this specific example, the medical doctor provides four different parameter values as known facts in combination with five general medical facts, like gender and age. Such known facts may be provided to the medical decision support system according to the present invention. One medical suggestion MSᵢ comprises all medical facts Fⱼ which influence said individual medical suggestion MSᵢ. For example, in the case of the follicle-stimulating hormone (FSH) value is influenced by the following medical facts: Gender, age, day of cycle, status of pregnancy and phase of life, like for example premenarchal, capable of reproduction, premenopausal, postmenopausal. These influencing factors are part of the definition of the medical suggestions MS₁ to MS₃₀₀ which are stored in a relational database, which is created by medical authors and experts. Also the corresponding weights and weights for combinations are comprised as will be explained in detail. The received known facts in form of values of medical facts Fⱼ, as defined in the independent claims, can be identified in the present example as follows:
● medical fact F₁ : age (in years): 27
● medical fact F₂: gender: female
● medical fact F₃: day of cycle: 6
● medical fact F₄: FSH(IU/1):7.5
● medical fact F₅: LH(IU/1): 16.9
● medical fact F₆: 17-ß-Östradiol (ng/l) 60.0
● medical fact F₇: HCG(IU/L): 0.5
● medical fact F₈: pregnancy, existence: no
● medical fact F₉: phase of life woman: capable of reproduction

In the selection process, all medical suggestions out of the 300 are selected for the subset S1 which do not comprise a knockout criterion which is fulfilled by the received known medical facts F₁ to F₉. In this embodiment, all medical suggestions are removed or i.e. are not selected for subset S1, which comprise the medical fact "pregnancy, existence: yes". Consequently, the basic set S0 is reduced to an amount of 150 medical suggestions during the selection of the subset S1. Furthermore, only those medical suggestions are taken to define S1, which comprise the values FSH = 7,5, LH = 16,9, or 17ß-Östradiol = 60 in the respective definition intervals. Consequently, all medical suggestions are ignored which do not comprise an association with the medical facts FSH, LH, or 17ß-Östradiol. Finally, the subset S1 comprises only a maximum of 50 medical suggestions out of the 300 of S0.

The database used for this particular example, comprises for/ associates with the medical suggestion *"FSH, within the reference region, not pregnant"* weights W_{i,j} for the respective associations/dependencies. Said weights of the database can be provided by medical experts during the generation of the database as has been described before. In the present Example 1 it is a relatively simple, binary evaluation. In case one of the following combinations, which may be seen as a combined requirement for several different medical facts, is true an individual score or 950 points is contributed to the corresponding combination. In this example, the combinations, i.e. the combined requirements, can be seen as distinct requirements, as only one requirement at a time can be true. Said combinations can be seen as several linked medical facts, which are linked or combined via Boolean operators like AND, OR, AND NOT, and any bracket combination thereof. In other words, the step of weighting a combination of a first medical fact and a second medical fact by applying a combination weight W_{i,combination} is carried out in this example 1. As the combinations presented in example 1 are distinct requirements, the sum of all scores for each combination can only be zero, in case none of the situations described by the combinations is true, or 950, in case one of said combinations is true. In other words, in case the requirement of the individual medical suggestion MSᵢ is fulfilled, 950 points are attributed to the said medical suggestion, otherwise 0 points are attributed. Several different requirements cannot be true at the same time, as for each combination only one true Boolean combination exists, the sum of the combinations of said medical suggestion finally is always zero or 950. Carrying out those combinations can be embodied very simple, for example first the gender is varied, then the day of cycle, and then the phase of life is varied. For the FSH values, a definition interval is provided. As a knockout criterion, the gender "male" is comprised. The database comprises for the medical suggestion *"FSH, within the reference region, not pregnant"* the following combinations of medical facts and interval:
- combination: gender=male AND age (>17-...) AND FSH(1.5-14.3)
- combination: gender=male AND age (>15-17) AND FSH(-13)
- combination: gender=female AND phase of life=capable of reproduction AND day of cycle (>0-2) AND FSH(5.49-8.23)
- combination: gender=female AND phase of life=capable of reproduction AND day of cycle (>2-4) AND FSH(6.2-9.3)
- **combination: gender=female AND phase of life=capable of reproduction AND day of cycle (>4-6) AND FSH(5.49-8.23)**

It should be noted that the previous combination is true for the given example and thus adds 950 points for this medical suggestion. Further used combinations are as follows:
- combination: gender=female AND phase of life=capable of reproduction AND day of cycle (>6-8) AND FSH(5.09-7.63)
- combination: gender=female AND phase of life=capable of reproduction AND day of cycle (>8-10) AND FSH(4.26-6. 39)
- combination: gender=female AND phase of life=capable of reproduction AND day of cycle (>10-12) AND FSH(3.98-5. 97)
- combination: gender=female AND phase of life=capable of reproduction AND day of cycle (>12-14) AND FSH(3.1-17.7)
- combination: gender=female AND phase of life=capable of reproduction AND day of cycle (>14-16) AND FSH(5.5-8.25)
- combination: gender=female AND phase of life=capable of reproduction AND day of cycle (>16-18) AND FSH(4.81-7.22)
- combination: gender=female AND phase of life=capable of reproduction AND day of cycle (>18-20) AND FSH(3.44-5.16)
- combination: gender=female AND phase of life=capable of reproduction AND day of cycle (>20-22) AND FSH(2.75-4.13)
- combination: gender=female AND phase of life=capable of reproduction AND day of cycle (>22-24) AND FSH(2.06-3. 09)
- combination: gender=female AND phase of life=capable of reproduction AND day of cycle (>24-26) AND FSH(3.44-5. 16)
- combination: gender=female AND phase of life=capable of reproduction AND day of cycle (>26-28) AND FSH(6.19-9.29)
- combination: gender=female AND phase of life=capable of reproduction AND day of cycle (>28-35) AND FSH(5.85-8.94)
- combination: gender=female AND phase of life=Postmenopausal AND FSH(23-116)

Moreover, as another example, for the medical suggestion MSᵢ *"FSH, above the reference region, not pregnant",* the following combinations of medical facts and intervals of definitions may be used in the database.
- combination: gender=male AND age (>17-...) AND FSH(14.3-20)
- combination: gender=male AND age (>15-17) AND FSH(13-..)
- combination: gender=male AND age (>11-14) AND FSH(4.6-..)
- combination: gender=female AND phase of life=capable of reproduction AND day of cycle (>0-2) AND FSH(8.23-12)
- combination: gender=female AND phase of life=capable of reproduction AND day of cycle (>2-4) AND FSH(9.3-12)
- combination: gender=female AND phase of life=capable of reproduction AND day of cycle (>4-6) AND FSH(8.23-11)
- combination: gender=female AND phase of life=capable of reproduction AND day of cycle (>6-8) AND FSH(7.63-10)
- combination: gender=female AND phase of life=capable of reproduction AND day of cycle (>8-10) AND FSH(6. 39-10)
- combination: gender=female AND phase of life=capable of reproduction AND day of cycle (>10-12) AND FSH(5. 97-10)
- combination: gender=female AND phase of life=capable of reproduction AND day of cycle (>12-14) AND FSH(17.7-25)
- combination: gender=female AND phase of life=capable of reproduction AND day of cycle (>14-16) AND FSH(8.25-14)
- combination: gender=female AND phase of life=capable of reproduction AND day of cycle (>16-18) AND FSH(7.22-14)
- combination: gender=female AND phase of life=capable of reproduction AND day of cycle (>18-20) AND FSH(5.16-12)
- combination: gender=female AND phase of life=capable of reproduction AND day of cycle (>20-22) AND FSH(4.13-10)
- combination: gender=female AND phase of life=capable of reproduction AND day of cycle (>22-24) AND FSH(3. 09-10)
- combination: gender=female AND phase of life=capable of reproduction AND day of cycle (>24-26) AND FSH(5. 16-12)
- combination: gender=female AND phase of life=capable of reproduction AND day of cycle (>26-28) AND FSH(9.29-12)
- combination: gender=female AND phase of life=capable of reproduction AND day of cycle (>28-35) AND FSH(8.94-11.5)
- combination: gender=female AND phase of life=postmenopausal AND FSH(116-150)

After summing up the single weights for each medical suggestion MSᵢ within the subset S1, the following result is provided:

| | |
|---|---|
| lab value FSH, strongly below the reference region, not pregnant | 0 Pts. |
| lab value FSH below the reference region, not pregnant | 0 Pts. |
| lab value FSH within the reference region, not pregnant | 950 Pts. |
| lab value FSH above the reference region, not pregnant | 0 Pts. |
| lab value FSH strongly above the reference region, not pregnant | 0 Pts. |
| lab value LH, strongly below the reference region, not pregnant | 0 Pts. |
| lab value LH below the reference region, not pregnant | 0 Pts. |
| lab value LH within the reference region, not pregnant | 0 Pts. |
| lab value LH above the reference region, not pregnant | 950 Pts. |
| lab value LH strongly above the reference region, not pregnant | 0 Pts. |
| lab value 17-ß-Östradiol, strongly below the reference region, not pregnant | 0 Pts. |
| lab value 17-ß-Östradiol below the reference region, not pregnant | 0 Pts. |
| lab value 17-ß-Östradiol within the reference region, not pregnant | 950 Pts. |
| lab value 17-ß-Östradiol above the reference region, not pregnant | 0 Pts. |
| lab value 17-ß-Östradiol strongly above the reference region, not pregnant | 0 Pts. |

This result can be sorted and medical suggestions below a predefined threshold, for example below 200 points, can be removed from the output. The resulting secondary output may be provided as follows:

| | |
|---|---|
| lab value **FSH** within the reference region, not pregnant | 950 Pts. |
| lab value **LH** above the reference region, not pregnant | 950 Pts. |
| lab value **17-ß-Östradiol** within the reference region, not pregnant | 950 Pts. |

As can be seen from the sorted result, the method and system provides the user with the information that in view of the given fact that the woman is not pregnant, it can be provided that the lab value FSH is within the reference region. Thereby, this result takes into account the previously described interrelations between the reference region and the day of cycle, and the age as well as the currently provided FSH value. The same holds true for the lab value LH and the lab value 17-ß-Östradiol. Thus, an automatic, computer-based and set oriented evaluation of the received known facts F₁-F₉ is presented.

### Practical supplements:

It might be an advantageous auxiliary measure to derive additional medical facts, for example in an automatic manner, and add those supplemented medical facts to the originally received known facts. This may be seen as supplementing the original input vector with supplemented medical facts. For example, medical facts F₈ and F₉ of the above described example can easily be derived automatically.

Therefore, the method and system of the present invention may be configured to generate new medical facts based on the received know facts. Either mathematical functions may be used, like for example for the body mass index, which is a 100% deterministic derivation or generation of new medical facts. But also medical facts can be generated which can be derived from the received known facts with a reasonable probability of correctness.

### Example 2: medical finding scenario for lab values

### Procedure:

The set of medical suggestions MSᵢ in this scenario comprises medical suggestions of the endocrinology including the medical suggestions of the lab value evaluation described in detail in example 1. In particular, the subset S0 of the example 2 comprises:
● diagnostic medical suggestions (for example PCO syndrom),
● plausibility medical suggestions (for example value of day of cycle and age of the patient are implausible),
● conclusion from medical suggestions (for example *hypergonadotropic, hyperandrogenemic ovarian insufficiency 1*),
● lab value evaluation medical suggestion (for example FSH, above the reference region, not pregnant),
● compatibility medical suggestion (for example compatible with a *hyperandrogenetic alopecia,*
● text block medical suggestion (for example *hyperandrogenaemia:* the term *"hyperandrogenaemia"* only states that one or more levels measured of androgen are above the reference region. Concerning interpretation of a level of androgen, it should be considered that the levels of an androgen vary during childhood, the phase of reproduction, during pregnancy, and in the postmenopause, different reference region ...), and
● auxiliary medical suggestions (for example constellations of symptoms, complexes of findings, etc.)

The medical doctor may instruct a laboratory, for example via an order form, to examine or evaluate specific lab values and the doctor expects as a result a detailed medical finding report in form of a letter. The method of the present invention and the system carrying out the method matches these needs and can provide the medical doctor with the respective response in different formats, such that the medical doctor may integrate the paper letter or the electronic letter into his IT management system of his medical practice/medical office or his clinic information system. If needed, the medical doctor, or any other user may add further medical facts to the already used received known facts.

In the following explicit example, a virtual patient *Tina Mustermann* may be provided for further explanations. The received known facts in form of values of medical facts Fⱼ, as defined in the independent claims, can be identified in the present example as follows:
Basic facts :
   ● F₁: age: 38
   ● F₂: gender: female (f)
Diagnostic specification (automatically received/inquired from the IT system of a medical practice)
   ● F₃: N64.3: Galaktorrhoe, not in connection with the birth and breastfeeding
   ● F₄: N91.1: secondary Amenorrhoe
Lab parameter:
   ● F₅: Prolactin (ng/ml): 51
   ● F₆: DHEAS (microgramm/ml): 5,3
   ● F₇: 17-OH-Progesteron (microgramm/ml): 0,5
   ● F₈: 17-ß-Estradiol (ng/l): 21
Clinical specification:
   ● F₉: visual field defect: yes
Derived facts:
   ● F₁₀: pregnancy, existence: no
   ● F₁₁: phase of life woman: capable of reproduction

### Different steps of example 2:

The basic set S0 of the present example can be seen as a subset of a more general basic set. S0 relates to gynecological endocrinology in combination with medical finding scenario for lab values. S0 contains ca. 1500 medical suggestions MSᵢ In a set operation, the subset S1 is selected. For this purpose, all medical suggestions MSᵢ out of S0 are selected, which at least comprise or are associated with one medical fact received by the user. In exemplary embodiments of the present invention age and gender may be seen as basic information, which should generally be applied to the system and may not be used as selection criteria, due to their general value. Based on the received input, the received known facts F₁ to F₁₁, 84 medical suggestions remain within the subset S1. The step of calculating a respective score for the suggestions MSᵢ of the subset S1 based on the received values of the medical facts Fⱼ and the respective weights W_{i,j} can be carried out in the following. As can be gathered from the following list, each medical suggestion is classified in a medical suggestion category, which is provided in brackets after the respective medical suggestion. If required, grouping of medical suggestions according to their affiliation of a category can be carried out.

### The calculation:

In the following calculations with a system of "classified probabilities" are described. As an exemplary embodiment, three classes may be used to evaluate the calculated score which may be seen as a probability. The class of suspicion, expressed by a score value between 200 and smaller than 600, the class of a probable assumption, with a score value larger or equal than 600 and below 950, and the class of highly probable assumption with a score value equal to or above 950 points. However, also less or more classes may be used, and also other limits for the respective calls can be used. It should be noted that there is a difference to a statistical probability, which is far distant from the herein used classified probabilities. The herein described scores for point values are always based on an individual case of consideration and evaluation, and is not based on population averaged evaluations. For example, for the individual case the statistically correct statement that 48 % of men older than 80 years suffer from an ischemic heart disease has no added value. Either the actually examined 85 year old man suffers from such a disease or does not suffer from said disease. It is impossible to suffer from that disease by an amount of 48 %. In other words, such statistical statements are only valuable if a group of persons is considered. Among 100 men above 80 years, approximately 48 of them suffer from said heart disease. However, this does not help any further for the individual case, when the individual case needs to be examined and analysed in detail. As will become apparent from and elucidated with explanations of the invention, the present invention makes use of such an individual case evaluation, although classified probabilities are used.

In the following list, the order of scores from high to low is presented to the user for the 48 remaining medical suggestions MSᵢ. This is the result list of the herein described individual case of Mrs. *Tina Mustermann.*

| **Medical Suggestion Name** | **Type** | **Points** |
|---|---|---|
| Exyclude intake of prolactin stimulating drugs and other causes of hyperprolactinemia | Activity | 1150 |
| Hyperprolactinemic amenorrhea | Diagnosis | 1018 |
| Secondary amenorrhea | Diagnosis | 975 |
| Evaluation of the hormonal pattern "prolactin level increased: 0.82 ("shadow polymer") | auxiliary Medical Suggestion | 950 |
| DHEA-sulphate (µg/ml) summarized > reference range | auxiliary Medical Suggestion | 950 |
| Auxiliary polymer- collection amenorrheae and oligomenorheae | auxiliary Medical Suggestion | 950 |
| Auxilliary polymer, invisible (bracket: androgen levels slightly elevated) | auxiliary Medical Suggestion | 950 |
| Pre-/postmenopasue beats amenorrhea | auxiliary Medical Suggestion | 950 |
| Testosterone (µg/l), below detection limit | auxiliary Medical Suggestion | 950 |
| 17-OH-progesterone (µg/l), Alert (phase of menstrual cycle, reproductive age, FM, pregnancy-existence, Tanner state) | Laboratory Value Assessment | 950 |
| 17ß-estradiol (ng/l)Alert (pregnancy- existence, pregnancy trimenon, week of pregnancy, phase of the cycle, Tanner stage, reproductive age, FM, amenorrhea) | Laboratory Value Assessment | 950 |
| DHEA-sulphate (µg/ml), above reference range | Laboratory Value Assessment | 950 |
| Prolactin (ng/ml), above reference range | Laboratory Value Assessment | 950 |
| Testosterone (µg/l), reference range | Laboratory Value Assessment | 950 |
| Reference range determination not possible, missing facts | Activity | 950 |
| Hyperandrogenemia | Conclusion | 950 |
| Hyperprolactinaemia | Conclusion | 950 |
| Secondary amenorrhea | Text module | 950 |
| Evaluation of the hormonal pattern "17-OH-progesterone level > 5 < 15 ng/ml for age < 50 years | Text module | 950 |
| Evaluation of the hormonal pattern "17ß-estradiol < 25 ng/l for age < 50 years": | Text module | 950 |
| Evaluation of the hormonal pattern "prolactin level increased and DHEA-sulphate level increased": 24.4 | Text module | 950 |
| Notice on galactorrhea | Text module | 950 |
| Hyperandrogenemia | Text module | 950 |
| Hyperprolactinaemia | Text module | 950 |
| homeostasis model assessment of insulin resistance [HOMA-IR) | Activity | 600 |
| Exclude metabolic syndrome | Activity | 400 |
| Calculate BMI | Activity | 400 |
| Recommendation: sonographic evaluation of ovaries and uterus | Activity | 400 |
| PCO-syndrome | Diagnosis | 301 |
| Nonclassical adrenal hyperplasia | Diagnosis | 272 |
| Consider bone density measurement | Activity | 251 |
| Microprolactinoma | Diagnosis | 239 |
| Exclude visual field disturbance | Activity | 201 |
| Consider or recommend MRT of pituitary region | Activity | 201 |
| Exclude hypothyroidism by measuring TSH | Activity | 200 |
| Exclude local pathology, if nipple discharge is detectable from only one mammary gland or one duct | Activity | 200 |
| estrogen deficiency | Conclusion | 200 |
| Investigate life style habits (sports, eating habits, weight reduction diet, psychological stress, interruption of normal day-night-rhythm). | Activity | 111 |
| Hypogonadotropic amenorrhea | Diagnosis | 101 |
| Hypothyroidism, symptoms (cluster) | Conclusion | 100 |
| Macroprolactinoma | Diagnosis | 92 |
| *hyperandrogenemic* ovarian insufficiency | Diagnosis | 77 |
| Female androgenetic alopecia | Diagnosis | 70 |
| Normogonadotropic amenorrhea | Diagnosis | 62 |
| Hypothalamic amenorrhea | Diagnosis | 61 |
| Hypergonadotropic hypogonadism | auxiliary Medical Suggestion | 57 |
| Hypogonadotropic hypogonadism | Diagnosis | 56 |
| Bulimia | Diagnosis | 55 |
| Pituitary amenorrhea | Diagnosis | 51 |
| Premature ovarian failure | Diagnosis | 51 |
| Asherman syndrome | Diagnosis | 50 |
| TSHoma | Diagnosis | 50 |
| Hyperthyroidism, symptoms (cluster) | Conclusion | 50 |
| Adiposity | Diagnosis | 40 |
| anorexia nervosa | Diagnosis | 40 |
| chronic renal failure | Diagnosis | 31 |
| endometriosis | Diagnosis | 21 |
| Turner syndrome | Diagnosis | 20 |
| cirrhosis | Diagnosis | 15 |
| hirsutism | Diagnosis | 11 |
| Mastitis nonpuerperalis | Diagnosis | 11 |
| Anovulatory cycle | Diagnosis | 11 |
| Corpus luteum insufficiency | Diagnosis | 10 |
| iatrogenic hyperthyroidism | Diagnosis | 10 |
| A high LH-FSH ratio and/or hyperandrogenemia are in favor of the suspected diagnosis of polycystic ovaries | Text module | 10 |
| 17ß-estradiol (ng/l), reference range | Laboratory Value Assessment | 6 |
| Autoimmune thyroiditis | Diagnosis | 5 |
| Riedel-thyroiditis | Diagnosis | 5 |
| Reifenstein syndrome | Diagnosis | 5 |
| contraceptive pill because of hormone constellaion? | Medication | 4 |
| Sheehan's syndrome | Diagnosis | -50 |
| 46,XX gonadal dysgenesis | Diagnosis | -114 |
| To consider: one of the subtypes of congenital adrenal hyperplasia, including heterozygosities | Diagnosis | -699 |

Moreover, in the following, a selected calculation example out of the above list is presented for illustrative purposes. In particular, the activity medical suggestion named "HOMA-IR", i.e. homeostasis model assessment of insulin resistance, is shown. In detail, the dependencies for this medical suggestion are provided in the following table. In the first column "name", respective medical facts and medical suggestions are provided. In the second column "type" it is indicated whether a medical suggestion or a medical fact is provided. In the third column "W_{i,j}", the values of the weight W_{i,j} for the calculation of the score is presented. In the last column "result, points", the respective calculated score of the respective medical suggestion or medical fact is presented.

Laboratory: homeostasis model assessment of insulin resistance [HOMA-IR] (Activity), Associations/Dependencies for this medical suggestion:

| **Name** | **Type** | **W_{i,j}** (a single value for Facts, 3 different values for the classified Probability for MS) | **Result Points** |
|---|---|---|---|
| PCO-syndrome | MS | Suspected (>=200-600):200 Pts. | 200 |
| | | Likely (>=600-950):200 Pts. | |
| | | Very Likely (>=950):200 Pts. | |
| | | → PCO does have a value from 301 Pts. for the described input facts of this example | |
| classical adrenal hyperplasia | MS | S:200,L:200,VL:200 → does have 0 Points | |
| nonclassical adrenal hyperplasia | MS | S:200,L:200,VL:200 → does have 272 Points | 200 |
| *hyperandrogenemic* ovarian insufficiency | MS | S:200,L:200,VL:200 → does have 0 Points | |
| BMI (kg/m²) | Fact | >29-.. : 200 Points | |
| acanthosis nigricans | Fact | Yes: 200 Points | |
| | | No: 0 Points | |
| Waist-Measurement | Fact | >80-..: 200 Points | |
| Waist-Hip-Ratio | Fact | >0,8-.. : 200 Points | |
| hirsutism | MS | S:200,L:200,VL:200 → does have 11 Points | 0 |
| Symptoms of an hyperandrogenaemia | Fact | Yes: 200 Points | |
| | | No: 0 Points | |
| alopecia androgenetica | Fact | Yes: 200 Points | |
| (existence) | | No: 0 Points | |
| clitoromegaly | Fact | Yes: 200 Points | |
| | | No: 0 Points | |
| testosterone (µg/l) | Fact | >0.8-..: 200 Points | |
| DHEAS (µg/ml) | Fact | >0.8-..: 200 Points → is 5.3 | 200 |
| **sum** | | | **600** |

As can be seen in this example of the medical suggestion HOMA-IR, this medical suggestion is associated with the medical suggestion PCO-syndrome. Consequently, when calculating the score of the HOMA-IR medical suggestion, also the score of the medical suggestion PCO-syndrome has to be calculated. The classification of probabilities with the classes suspected (S), likely (L), and very likely (VL), is used in this example. For clarity reasons, the underlying calculation systematic and structure of the PCO-syndrome are not presented. However, for the given facts of the example of *Tina Mustermann,* calculated value or score of the PCO-syndrome is 301. According to the weight, 200 points are attributed to the PCO-syndrome, which are shown in the first row and the last column. Furthermore, the medical fact of the body mass index is shown within the sixth row of the table. 200 points are attributed to the HOMA-IR medical suggestion in case the BMI is larger than 29. However, as in the present example, no BMI value is provided, no points are attributed in the respective column. In a similar fashion, the remaining medical suggestions MSᵢ and medical facts Fⱼ of the HOMA-IR medical suggestions are calculated such that the sum of 600 is the result of the score.

The method and system of the present invention are configured in this embodiment of Example 2 to generate a letter, based on the previously explained calculations and a general letter template, which can be comprised by the database, which looks as follows:

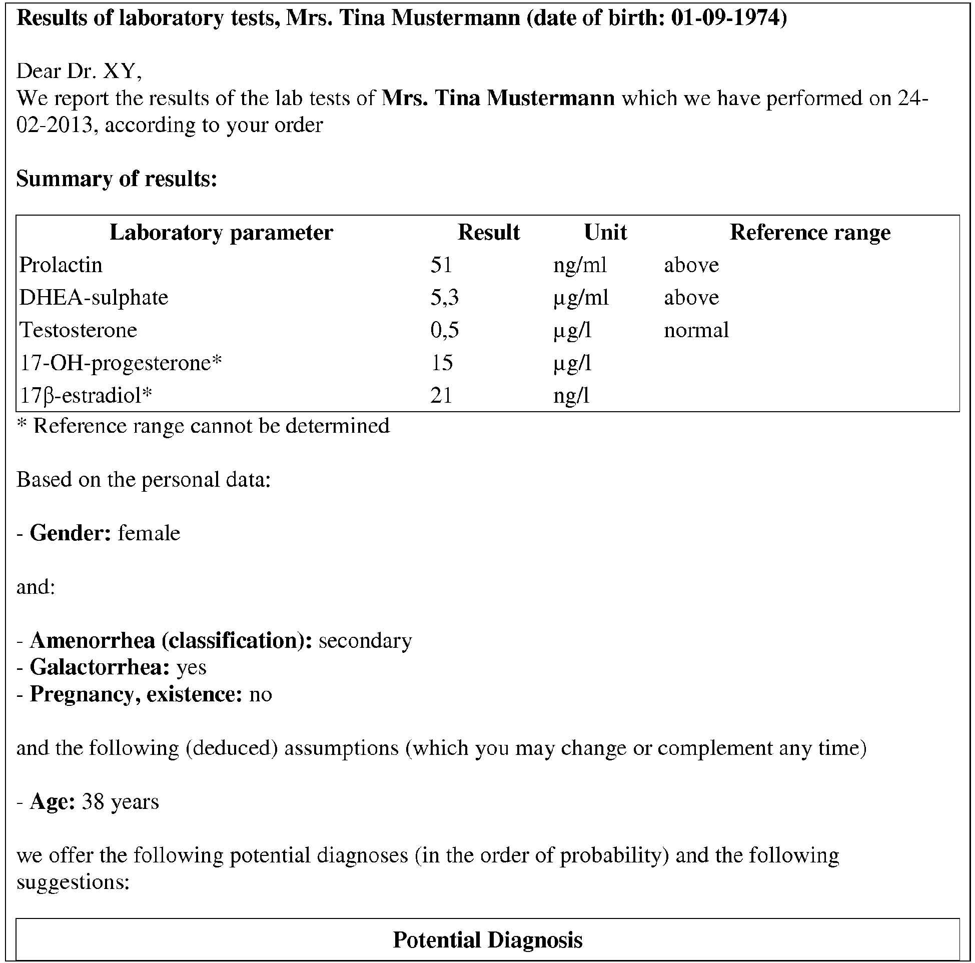

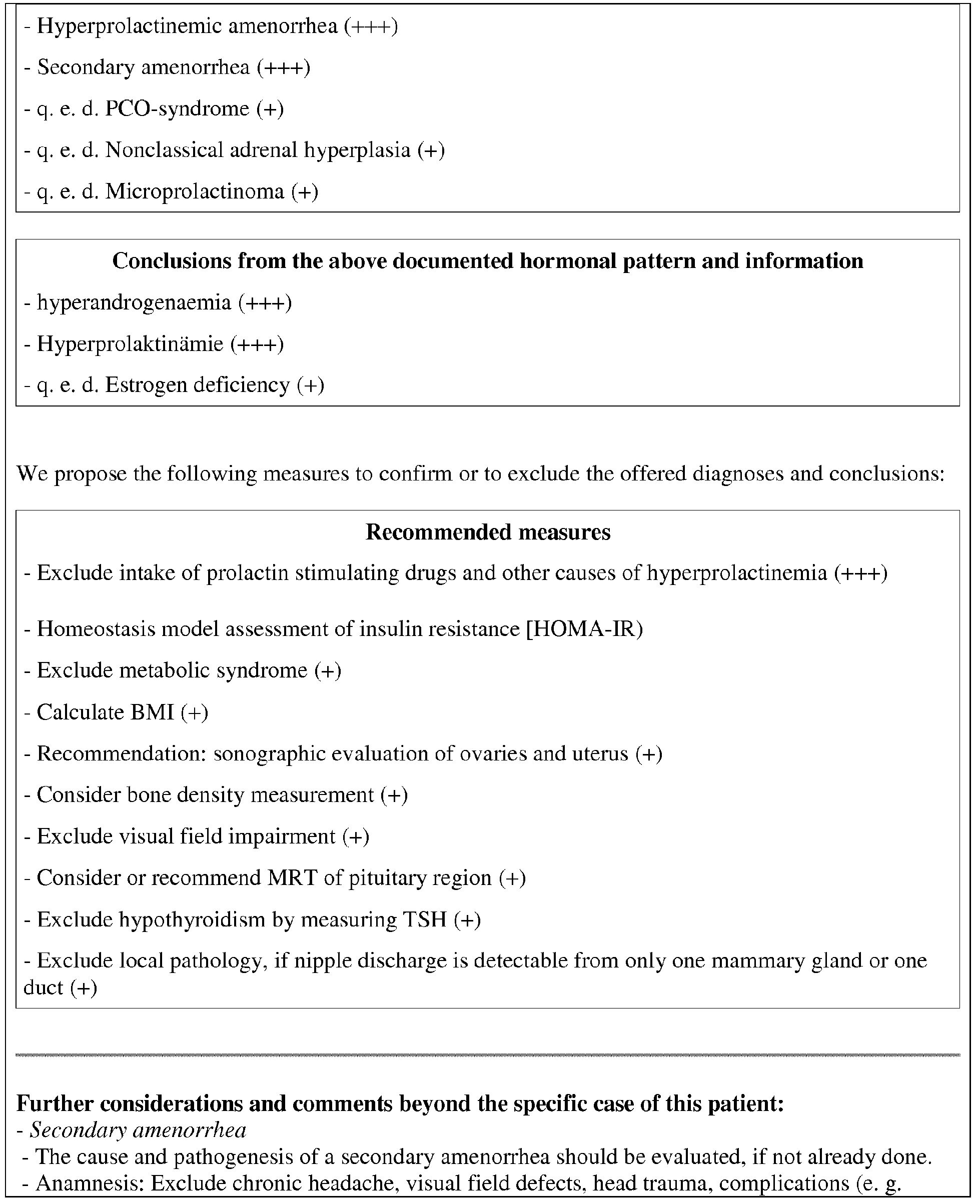

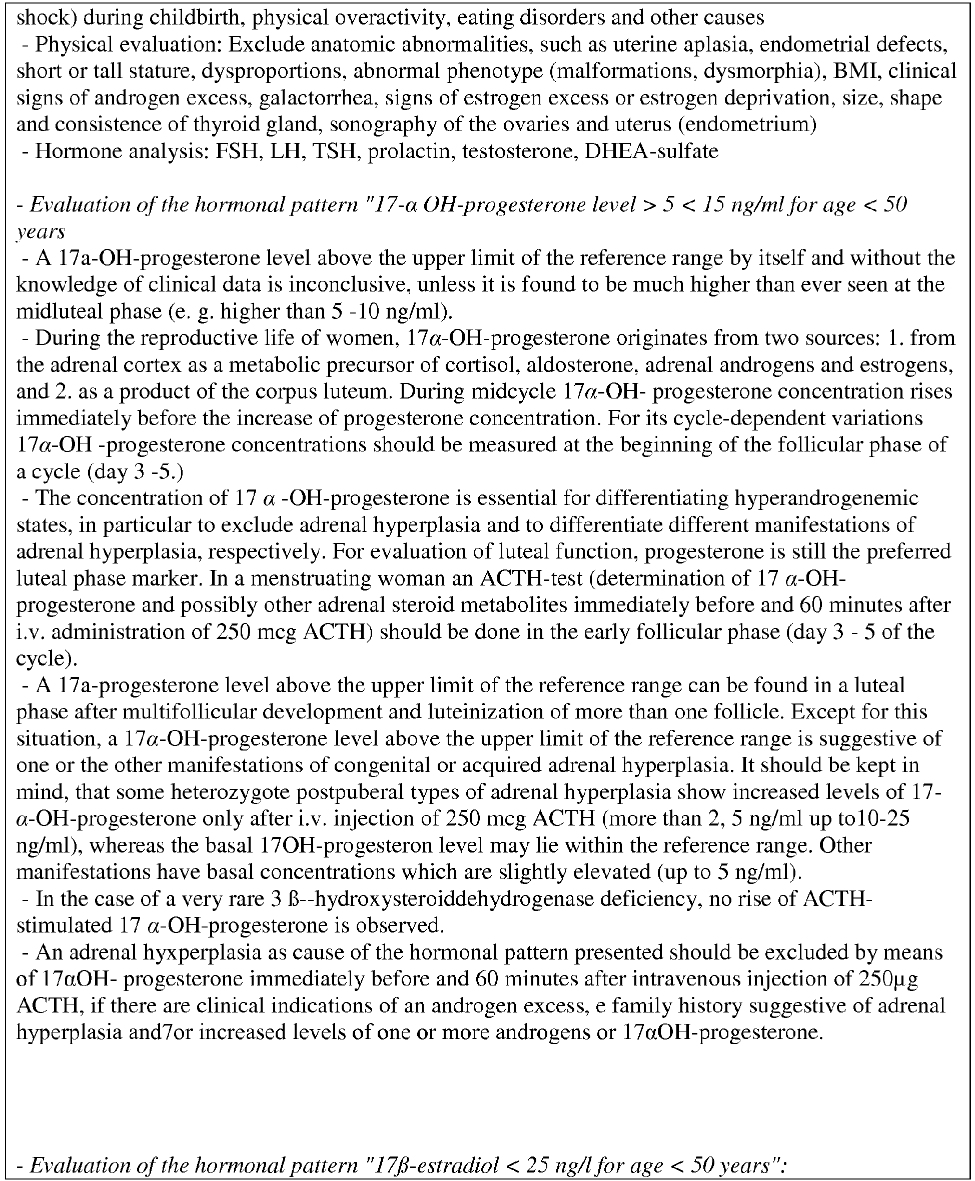

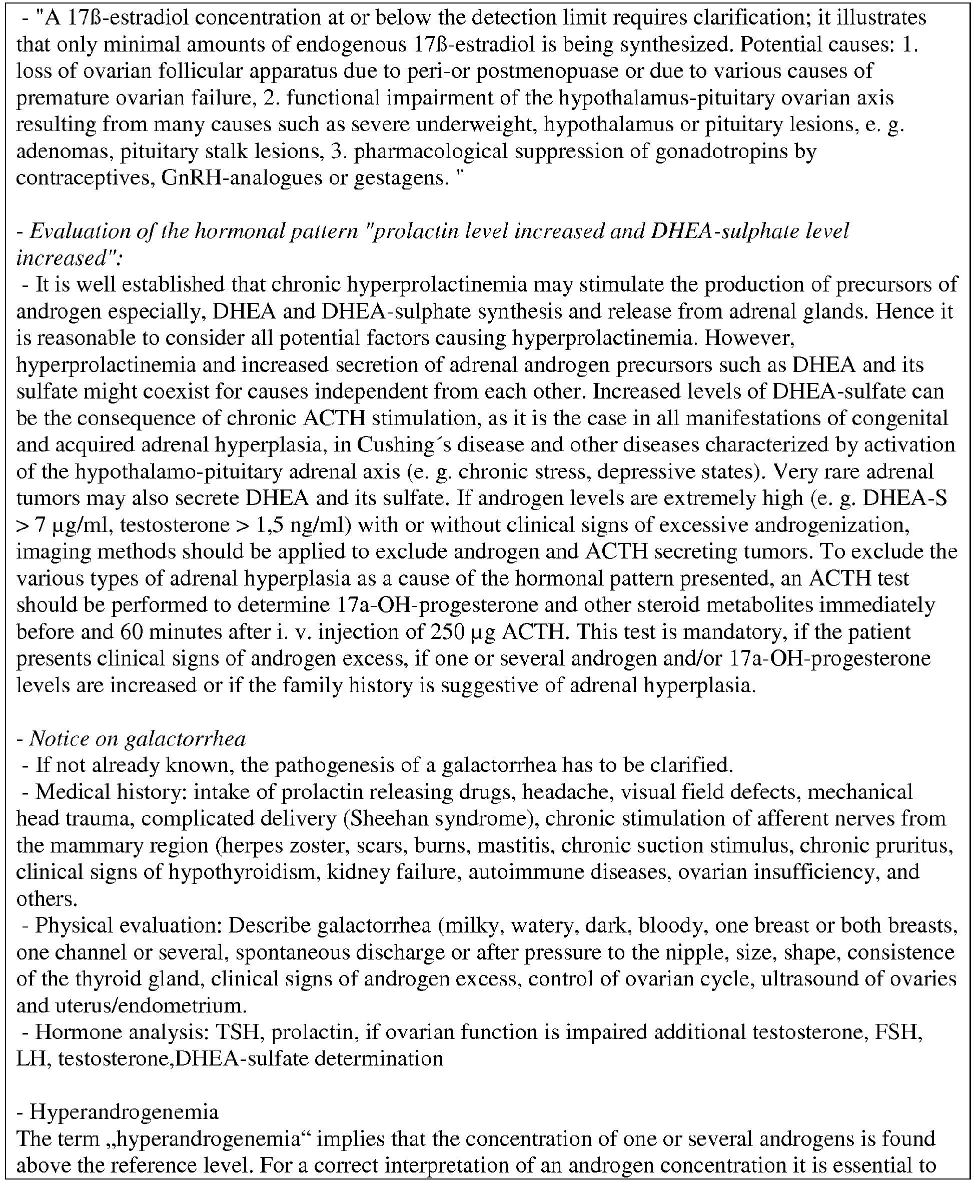

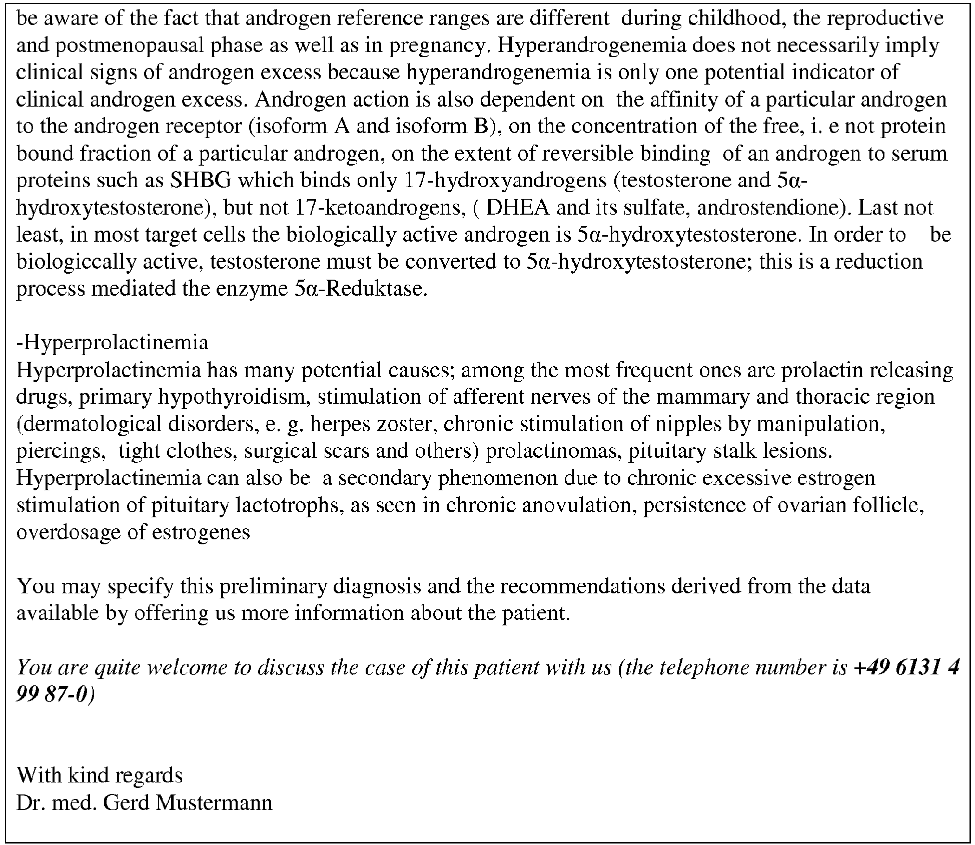

## Claims

1. Method of generating a medical suggestion useful for supporting a process of medical decision making, the method comprising the steps
providing for a database with a basic set S0 of medical suggestions MSᵢ (step 1)
wherein in the database at least some of the medical suggestions are associated with at least one respective medical fact Fⱼ,
wherein in the database the respective medical fact Fⱼ of the at least some medical suggestion MSᵢ is associated with a weight W_{i,j},
the method further comprising the step
receiving known facts in the form of values of medical facts Fⱼ, which known facts are associated with an individual patient (step 2),
selecting a subset S1 of medical suggestions out of the basic set S0 based on the received known facts (step 3), and
calculating a respective score for at least some medical suggestions MSᵢ of the subset S1 based on the received values of the medical facts Fⱼ and the respective weight W_{i,j} (step 4).

2. Method according to claim 1,
wherein the subset S1 is **characterized in that** the medical suggestions comprised in S1 are associated with medical facts for which values were received as known facts.

3. Method according to one of the preceding claims,
the method further comprising the step
assessing each medical suggestion of the basic set S0 upon the step of selecting the subset S1.

4. Method according to one of the preceding claims
wherein the step of selecting the subset S1 is processed on a set basis.

5. Method according to one of the preceding claims
wherein the medical suggestions MSᵢ are respectively embodied as an element chosen from the group comprising a medical diagnosis, a medical finding, a medication, an anamnesis, an auxiliary suggestion, an evaluation of a lab value, a medical plausibility, a medical conclusion, a medical measure, a medical instruction, a medical statement, a medical question, symptoms, a cluster of symptoms, a text block, a nutrition suggestion, a fitness suggestion, a care suggestion, a rehab suggestion, a genetic aspect, a histology finding, a physiological process, a finding out of a patho-physiological process, a quality indicator, a treatment recommendation, a therapy recommendation, a process suggestion, a medical investigation suggestion, a patient questionnaire, a professional questionnaire, and any combination thereof.

6. Method according one of the preceding claims,
wherein each respective score of the medical suggestions MSᵢ of the subset S1 represents a probability that the respective medical suggestion is correct.

7. Method according to one of the preceding claims,
wherein the medical facts Fⱼ are respectively embodied as an element chosen from the group comprising an age of the patient, a gender of the patient, a body weight of the patient, a body height of the patient, a physiological parameter, a biological parameter, a chemical parameter, a medical parameter, a symptom, an information associated with a medical complaint, a result of a medical finding, information associated with living conditions of the patient, information about the patient which is useful for describing a medical situation of the patient, a diagnosis, medical data, medication data, fitness data, nutrition data, rehab data, care data, telemetry data, statistical data, medical reference data, an anamnesis, a risk factor, an allergy, a habitat of the patient, a job situation of the patient, a housing situation of the patient, imaging data, regional weather data, regional environmental data, endemic data, epidemic data, a result of a function test, information received from a professional or the patient via a questionnaire and any combination thereof.

8. Method according to one of the preceding claims,
wherein the step of calculating the score of medical suggestions comprises the steps
weighting a received first value of a first medical fact Fⱼ of a first medical suggestion MSᵢ by applying a first weight W_{i,j} resulting in a first suggestion result,
weighting a received second value of a second medical fact Fₖ of the first medical suggestion MSᵢ by applying a second weight W_{i,k} resulting in a second suggestion result, and
summing the first suggestion result and the second suggestion result to the score of the suggestion MSᵢ.

9. Method according to claim 8, the method further comprising the step
weighting a combination of the first medical fact Fⱼ and the second medical fact Fₖ by applying a combination weight W_{i, combination j-k}, and
wherein the combination is chosen from the group of Boolean combinations comprising AND, OR, AND NOT, and any bracket combination thereof.

10. Method according to one of the claims 8 or 9, the method further comprising the step
repeating the steps of claim 8 or 9 with respect to at least one second medical suggestion Sₘ.

11. Method according to one of the preceding claims,
wherein the weight W_{i,j} is a probability distribution which expresses the probability that the corresponding medical suggestion MSᵢ is correct based on a value of the medical fact Fⱼ.

12. Method according to one of the preceding claims,
wherein the step of calculating the score of medical suggestions comprises the steps
receiving a first value of a first medical fact Fⱼ of a first medical suggestion Sᵢ,
receiving a second value of a second medical fact Fₖ of the first medical suggestion Sᵢ, and
weighting a combination of the first medical fact Fⱼ and the second medical fact Fₖ by applying a combination weight W_{i, combination j-k}.

13. Method according to claim 12,
wherein the weight W_{i,} combination ⱼ₋ₖ.is a probability distribution which expresses the probability that the corresponding medical suggestion MSᵢ is correct based on the combination of values of the medical facts Fⱼ and Fₖ.

14. Method according to one of the preceding claims,
wherein the database provides for a structure such that all medical facts Fⱼ are autarkic and equivalent.

15. Method according to one of the preceding claims, the method further comprising the steps
ranking the medical suggestions MSᵢ of the subset S1 in an order of their respective calculated score, and
providing the order of ranked medical suggestions to the user.

16. Method according to one of the preceding claims, the method further comprising the steps
supplementing the received known facts by at least one medical suggestion out of the subset S1 based on the respective calculated score of said at least one medical suggestion, and
repeating step 2, step 3 and step 4 with the supplemented received known facts.

17. Method according to claim 16, further comprising the steps
providing for a score threshold, and
selecting the at least one medical suggestion out of the subset S1 if the score of said medical suggestion is larger than the score threshold.

18. Method according to one of the preceding claims, the method further comprising the steps
classifying the individual received known facts with respect to the respective creator of said received known facts, and
applying a prioritization of the received known facts based on the respective creator.

19. Method according to one of the preceding claims,
wherein at least one of the received medical facts Fⱼ is provided in form of a time evolvement, and
wherein said time evolvement is represented by a vector comprising n values of the medical fact at n different points in time.

20. Method according to one of the preceding claims,
wherein the basic set S0 comprises a plurality of medical suggestions which are respectively embodied as diagnosis,
wherein each diagnoses is associated with medical facts which are embodied as a symptom or as a medical fact which is relevant for or is associated with said diagnosis.

21. Method according to one of the preceding claims,
wherein a first medical suggestion of the basic set S0 is associated with a second medical suggestion such that during the step of calculating the score of the first medical suggestion a score of the second medical suggestion is calculated.

22. Method according to one of the preceding claims,
wherein at least one medical suggestion of the basic set S0 is associated with a plurality of medical facts Fⱼ, and
wherein at least a part of the plurality of medical facts are thematically linked together in the database to form a group of medical fact.

23. Method according to one of the preceding claims,
wherein in said database at least one medical suggestion MSᵢ is associated with a knock out criterion for said medical suggestion, and
wherein said at least one medical suggestion is not selected for the subset S1 in case the received known facts fulfill said knock out criterion.

24. Method according to one of the preceding claims,
wherein in the database at least one medical suggestion MSᵢ is associated with a must have criterion for said medical suggestion, and
wherein said at least one medical suggestion is only selected for the subset S1 if the received known facts fulfill said must have criterion.

25. Method according to one of the preceding claims, the method further comprising the step
generating an output based on the calculated scores, and
wherein the output is chosen from the group comprising a list of probable medical suggestions ranked in the order of the respective calculated score, a report, a letter addressed to the patient, a letter addressed to lab, a letter addressed to a lab comprising an instruction or suggestion for a further measurement in said lab, a medical finding letter, a medical finding letter with a sender identification of a clinician, a question or a question set to a user, a question to a user in form of a graphical interface, an order, an order of a medicament, and any combination thereof.

26. Method according to claim 25, the method further comprising the step
providing said generated output to the user in form of output data,
receiving amendment information about an amendment of the output data caused by the user, and
adapting said database based on the received amendment information.

27. Method according to claim 26,
wherein the adaption of the database is chosen from the group comprising
adapting an association of at least one medical suggestions with at least one respective medical fact Fⱼ, adapting at least one weight W_{i,j},adapting selection rules for selecting the subset S1 of medical suggestions out of the basic set S0, and any combination thereof.

28. Method according to one of the preceding claims,
wherein the steps of selecting the subset S1 and of calculating the scores is carried out by a calculation unit,
the method further comprising the step
providing for an interface between the calculation unit and a medical fact source for facilitating data transmission between the database and the medical fact source, and
wherein the interface is configured to facilitate transmission of known facts in the form of values of medical facts Fⱼ of at least one individual patient to the calculation unit when the medical fact source is connected to the interface.

29. Method according to claim 28, wherein calculation unit is configured to process said known facts received from the medical fact source, and
wherein the medical fact source is chosen from the group comprising a sensor, a sensor for determining a physiological parameter, a smartphone with sensor data, a data system of a medical practice, a data system of a hospital, a data system of a care or reha organization, a network of a medical office and/or hospitals/a care or reha organization or a network of integrated care, a database of a medical office, a database of a hospital/care or reha organization, a database of national medical networks , a software providing structured medical, environmental or statistical data, a lab device, and a lab robot.

30. A program element for generating a medical suggestion useful for supporting a process of medical decision making which program element, when being executed by a processor, is adapted to carry out:
making a database available with a basic set S0 of medical suggestions sᵢ,
wherein in the database at least some of the medical suggestions are associated with at least one respective medical fact Fⱼ,
wherein in the database the respective medical fact Fⱼ of a medical suggestion MSᵢ is associated with a weight W_{i,j},
wherein the program element is further adapted to carry out
receiving known facts in the form of values of medical facts Fⱼ, which known facts are associated with an individual patient,
selecting a subset S1 of medical suggestions out of the basic set S0 based on the received known facts, and
calculating a respective score for at least some medical suggestions MSᵢ of the subset S1 based on the received values of the medical facts Fⱼ and the respective weight W_{i,j}.

31. A computer-readable medium, in which a computer program for generating a medical suggestion useful for supporting a process of medical decision making is stored, which computer program, when being executed by a processor, is adapted to carry out:
making a database available with a basic set S0 of medical suggestions sᵢ,
wherein in the database at least some of the medical suggestions are associated with at least one respective medical fact Fⱼ,
wherein in the database the respective medical fact Fⱼ of a medical suggestion MSᵢ is associated with a weight W_{i,j},
wherein the program element is further adapted to carry out
receiving known facts in the form of values of medical facts Fⱼ, which known facts are associated with an individual patient,
selecting a subset S1 of medical suggestions out of the basic set S0 based on the received known facts, and
calculating a respective score for at least some medical suggestions MSᵢ of the subset S1 based on the received values of the medical facts Fⱼ and the respective weight W_{i,j}.

32. Medical decision support system for generating a medical suggestion useful for supporting a process of medical decision making,
the system comprising
database with a basic set S0 of medical suggestions Sᵢ,
wherein in the database at least some of the medical suggestions are associated with at least one respective medical fact Fⱼ,
wherein in the database the respective medical fact Fⱼ of a medical suggestion MSᵢ is associated with a weight W_{i,j},
a receiving apparatus for receiving known facts in the form of values of medical facts Fⱼ, which known facts are associated with an individual patient,
a calculation unit configured for selecting a subset S1 of medical suggestions MSᵢ out of the basic set S0 based on the received known facts, and
wherein the calculation unit is configured for calculating a respective score for at least some medical suggestions MSᵢ of the subset S1 based on the received values of the medical facts Fⱼ and the respective weight W_{i,j}.
